# EUROPEAN PATENT SPECIFICATION

(11) **EP 0 413 289 B1**
(45) Date of publication and mention of the grant of the patent: **01.02.1995**
(21) Application number: 90115525.9
(22) Date of filing: 13.08.1990
(51) Int. Cl.: C07D 413/12, A61K 31/41, C07D 261/08

(54) **Oxadiazolyl-phenoxyalkylisoxazoles and their use as antiviral agents**
Oxadiazolyl-Phenoxyalkyl-Isoxazole und ihre Verwendung als antivirale Mittel
Oxadiazolyl-phenoxyalkyl-isoxazoles et leur utilisation comme agents antiviraux

(30) Priority: 18.08.1989 US 396419; 21.08.1989 US 396414; 04.06.1990 US 532480; 07.06.1990 US 534917
(43) Date of publication of application: 20.02.1991
(73) Proprietor: STERLING WINTHROP INC., New York, NY 10016 (US)
(72) Inventor: Diana, Guy Dominic, East Nassau, New York (US); Bailey, Thomas Robert, Glenmont, New York (US)
(74) Representative: Baillie, Iain Cameron

(56) References cited:
- EP-A- 197 282
- EP-A- 0 111 345
- EP-A- 0 137 242
- EP-A- 0 207 453
- EP-A- 0 207 454
- DE-A- 2 722 331
- GB-A- 1 023 383
- US-A- 4 942 241
- US-A- 4 945 164
- Chemical Pharm. Bull., 21, 1641-50 (1973)

## Description

### BACKGROUND OF THE INVENTION

### a) Field of the Invention

This invention relates to novel heterocyclic substituted-phenoxyalkylisoxazoles and -furans, to methods for the preparation thereof, and compositions and methods for the use thereof as antiviral agents.

### b) Information Disclosure Statement

Diana U.S. Pat. 4,451,476, issued May 29, 1984, discloses antivirally active compounds having the formula 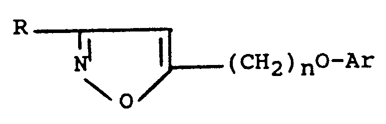
wherein:
R is alkyl of 1 to 3 carbon atoms;
n is an integer from 4 to 8; and
Ar is phenyl or phenyl substituted by one or two substituents selected from the group consisting of halogen, lower-alkyl, lower-alkoxy, nitro, cyano, carboxy, lower-alkoxycarbonyl, lower-alkanoyl, 1-oximino-lower-alkyl, hydrazinocarbonyl, carbamyl and N,N-di-lower-alkylcarbamyl.

Sterling Drug Inc. European Patent Application Publ. No. 137,242, published April 17, 1985, discloses antivirally active compounds having the formula 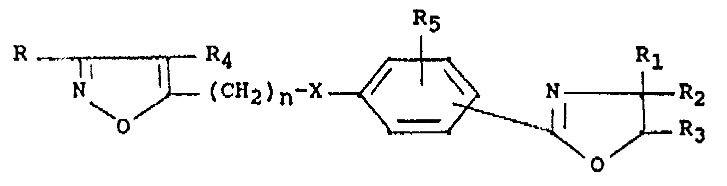
wherein:
R, R₁, R₂, R₃, and R₄ are each hydrogen or alkyl of 1 to 3 carbon atoms optionally substituted by hydroxy lower-alkanoyloxy, lower-alkoxy, chloro, or N=Z, wherein N=Z is amino, lower-alkanoylamino, lower-alkylamino, di-lower-alkylamino, 1-pyrrolidinyl, 1-piperidinyl or 4-morpholinyl; with the proviso that R is other than hydrogen;
R₅ is hydrogen, lower-alkyl, halogen, nitro, lower-alkoxy, lower-alkylthio or trifluoromethyl;
X is 0 or a single bond; and
n is an integer from 3 to 9;
and to pharmaceutically acceptable acid-addition salts thereof.

Sterling Drug Inc. European Patent Application Publ. No, 207,453, published January 7, 1987, discloses compounds of Formula I below where Het is 1,3,4-oxadiazol-5-yl, and contemplates compounds where Het is 1,2,4-oxadiazol-5-yl.

European Patent Publication 207,453 describes antiviral compounds of the formula 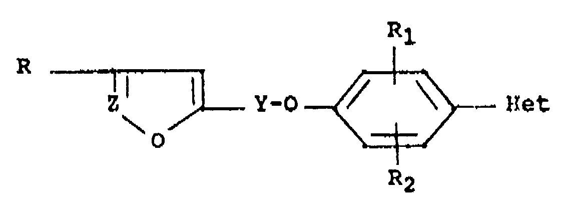
wherein Y is alkylene of 3-9 carbon atoms, Z is N or HC, R is hydrogen or lower alkyl of 1-3 carbon atoms, provided that if Z is N, R is lower alkyl, R₁ and R₂ are each hydrogen, halogen, methyl, nitro, lower alkoxy carbonyl, or trifluoromethyl and Het is a heterocyclic group selected from a defined set of alternatives but not including the specific substituted oxadiazole groups of the present invention.

European Patent Publication 137,242 describes compounds similar to those of Publication 207,453 where Z is restricted to nitrogen and the heterocyclic group is a 2-oxazolyl group.

### SUMMARY OF THE INVENTION

The present invention relates to compounds of the formula 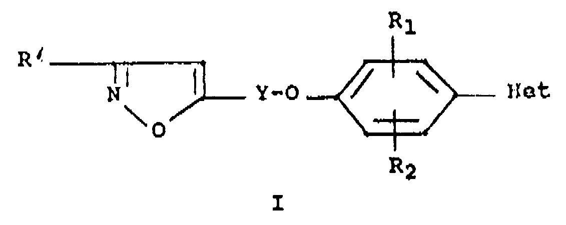
wherein:
Het is selected from 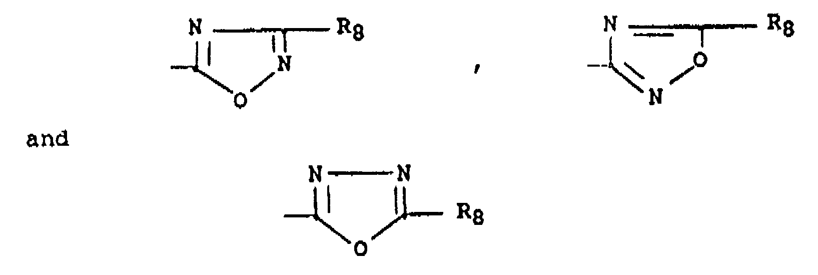
Y is an alkylene bridge of 3-9 carbon atoms;
R' is alkyl of 1-5 carbon atoms, or hydroxyalkyl of 1-5 carbon atoms;
R₁ and R₂ are hydrogen, halogen, C₁₋₅ alkyl, C₁₋₅ alkoxy, nitro, C₁₋₅ alkoxycarbonyl or trifluoromethyl; and
R₈ is alkyl of 1-5 carbon atoms;
or pharmaceutically acceptable acid-addition salts of basic members thereof.

A preferred class of compounds within the scope of Formula I are those of the formula 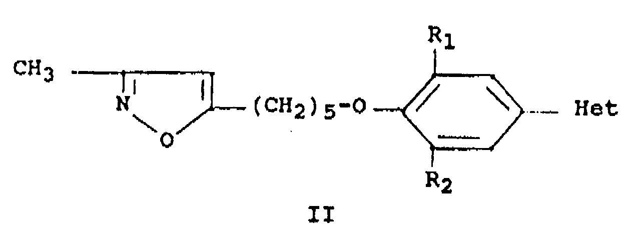
The invention also relates to compositions for combating viruses comprising an antivirally effective amount of a compound of Formulas I and II in admixture with a suitable carrier or diluent, and to methods of combating viruses therewith, including the systemic treatment of viral infections in a mammialian host.

### DETAILED DESCRIPTION INCLUSIVE OF PREFERRED EMBODIMENTS

The compounds of Formula I where Het is a nitrogen-containing heterocyclic group are sufficiently basic to form stable acid-addition salts with strong acids, and said salts are within the purview of the invention. The nature of the acid-addition salt is immaterial, provided it is derived from an acid the anion of which is essentially non-toxic to animal organisms. Examples of appropriate acid-addition salts include the hydrochloride, hydrobromide, sulfate, acid sulfate, maleate, citrate, tartrate, methanesulfonate, p-toluenesulfonate, dodecyl sulfate, cyclohexanesulfamate, and the like.

When the term halogen is used to define the substituents R₁ and R₂, any of the four common halogens, fluorine, chlorine, bromine or iodine are contemplated; the terms lower alkyl and lower alkoxy refer to groups having 1-5 carbon atoms and the term lower-alkoxycarbonyl refers to such groups having from two to four carbon atoms.

The compounds of Formula I can be prepared by a process which comprises reacting a compound of the formula 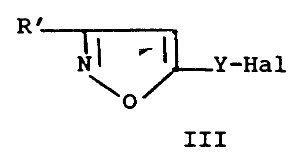
wherein Hal is chlorine, bromine or iodine, with an alkali metal salt of a compound of the formula 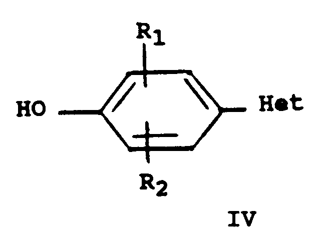
The compounds of Formula I can also be prepared by an alternative process which comprises reacting a compound of the formula 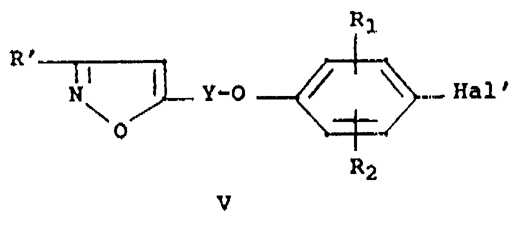
where Hal' is bromine or iodine, with a compound of the formula

(R'')₃Sn-Het' VI

where R'' is alkyl of 1-6 carbon atoms, and Het' is any of the aromatic type heterocyclic groups included in the definition of Het in Formula I; in the presence of a palladium complex catalyst.

The process for the preparation of compounds of Formula I by reacting intermediates of Formulas III and IV takes place by heating the reactants in an inert solvent in the presence of an alkali metal base, e.g., potassium carbonate or potassium hydroxide at a temperature between about 50°C. and 150°C.

The intermediates of Formula III are prepared by reacting an alkali metal derivative of an isoxazole of the formula 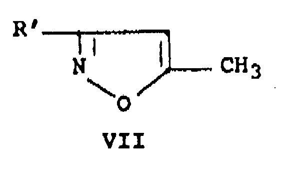
with a dihalide, Hal-Y'-Hal, where Y' is an alkylene bridge of 2 to 8 carbon atoms. Said alkali metal derivative is prepared in situ by treating the compound of Formula VII with an organo-alkali metal base under anhydrous conditions. Preferred organo-alkali metal bases are butyllithium and lithium diisopropylamide.

The intermediates of Formula IV are a generically known class of heterocyclic substituted phenols, prepared as described hereinafter in the general description and specific examples.

In the alternative process comprising reacting compounds of Formulas V and VI, the process is carried out using approximately equimolar amounts of the reactants in an inert solvent at a temperature between about 50°C. and 100°C., conveniently at the reflux temperature of the solvent. The reaction is complete in a period ranging from 5-24 hours. The palladium complex catalyst, present to the extent of about 5 mole percent, can be any such catalyst known to effect cross-coupling of organotin compounds with organic halides [cf. Kosugi et al., Bull. Chem. Soc. Japan 59, 677-679 (1986)], for example PdCl₂-(PPh₃)₂, Pd(PPh₃)₄, PdCl₂[P(o-tolyl)₃]₂, PdCl₂+2P(OEt)₃ and PdCl₂(PhCN)₂. A preferred catalyst is dichlorobis-(triphenylphosphine)palladium [PdCl₂(PPh₃)₂].

The intermediates of Formula V are prepared by reacting an alkali metal salt of a phenol of the formula 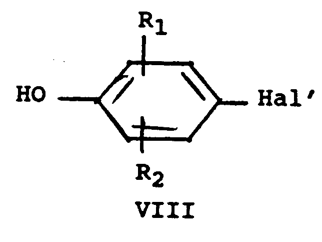
with a compound of Formula III in a procedure analogous to that of the reaction of III with IV.

The organotin reagent of Formula VI is prepared by known procedures comprising reacting a tri-lower-alkyl-tin halide with an unsubstituted aromatic heterocycle in the presence of a strong base such as butyllithium under anhydrous conditions. The trialkyltin moiety enters the most reactive position on the heterocyclic ring; however, the trialkyltin moiety can be directed to other positions on the heterocyclic ring by using the appropriate halo-substituted heterocycle.

Certain compounds of the invention can be prepared by construction of the Het ring from intermediates having a cyano or amide group on the phenyl ring, as follows.

The invention contemplates compounds of the formulas 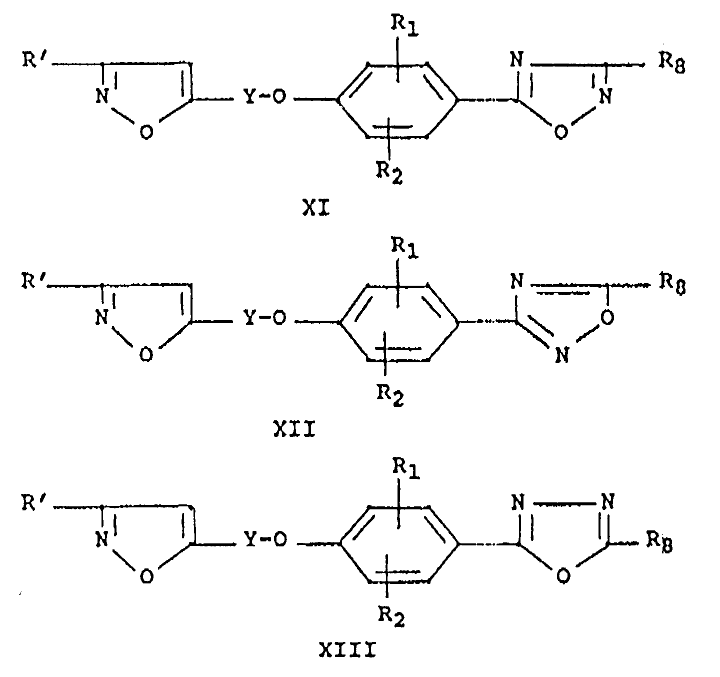
where R' is C₁₋₅-alkyl or hydroxy-alkyl of 1-5 carbon atoms, R₈ is alkyl of 1-5 carbon atoms, and Y₁ R₁ and R₂ have the meanings given hereinabove.

The compounds of Formula XI can be prepared by reacting an amide of Formula XIV (below) with a compound of the formula (CH₃)₂N-C(OCH₃)₂-R₈, followed by reaction of the resulting product with hydroxylamine in acetic acid solution 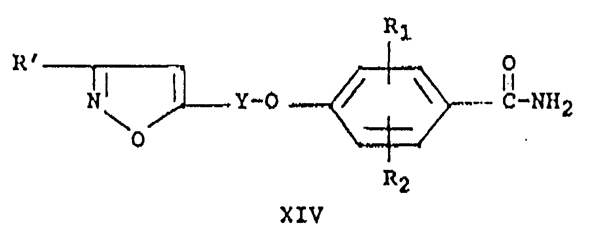
The amides of Formula XIV are in turn prepared by acid hydrolysis of the corresponding nitrile 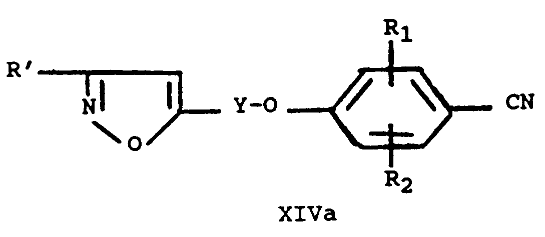
The compounds of Formula XII can be prepared by reacting a nitrile of Formula IX, or the imino chloride derivative thereof, with hydroxylamine, followed by reaction of the resulting product with an anhydride of the formula (R₈CO)₂O.

Alternatively, the compounds of Formulas XI and XII can be prepared by reacting a compound of one of the formulas: 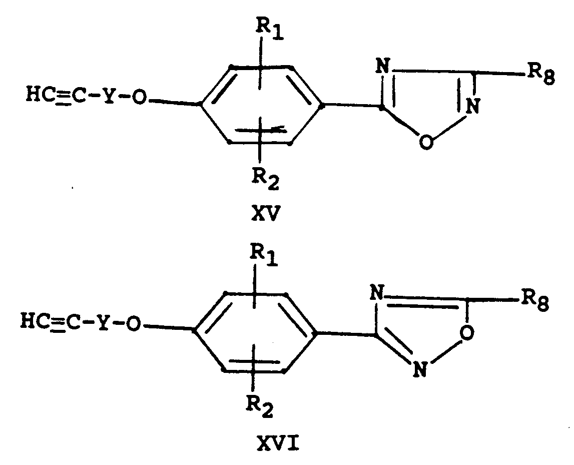
with a 1-nitroalkane (R′CH₂NO₂) and phenylisocyanate or with an alkanecarboxaldehyde oxime (R′CH=NOH) and N-halosuccinimide where halo is bromo or chloro. The intermediates of Formulas XV and XVI are in turn prepared by procedures described hereinabove and as illustrated in Example 5 below.

The compounds of Formula XIII are prepared by reacting a compound of Formula III with a compound of Formula IV where Het is 5-C₁₋₅-alkyl-1,3,4-oxadiazol-2-yl. The phenolic compounds of Formula IV are prepared as described in U.S. Patent 4,218,458, issued August 19, 1980.

Alternatively, the compounds of Formula XIII can be prepared by cyclization of compounds of the formula 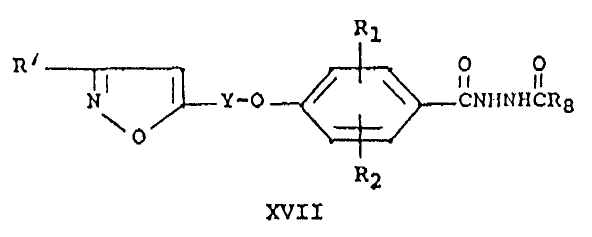
by reaction with hexamethyldisilazane in the presence of tetrabutylammonium trifluoride according to the method of Rigo et al., Synthetic Communications 16, 1665-9 (1986). The intermediate acylhydrazines of Formula XVII are prepared from the corresponding carboxylic acids: 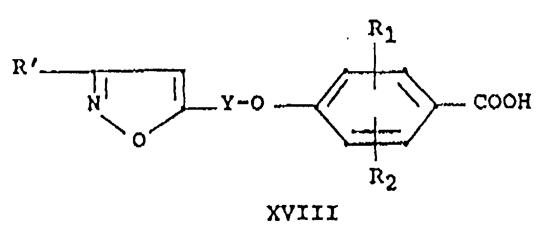
by conventional amide formation. The acid XVIII is conveniently prepared by hydrolysis of the nitrile of Formula XIVa.

The compounds of Formula XIII where R' is alkyl of 1-5 carbon atoms can also be prepared from the compounds of Formula I where Het is 1H-tetrazol-5-yl by reaction with an alkanoic acid halide, R₈CO-Hal'' in where Hal'' is chlorine or bromine, or an alkanoic acid anhydride, (R₈CO)₂O.

The structures of the compounds of the invention were established by the modes of synthesis, by elementary analysis, and by infrared and nuclear magnetic resonance spectra.

The following examples will further illustrate the invention.

### Example 1 (Comparative)

### 3-Methyl-5-{5-[4-(1,3,4-oxadiazol-2-yl)phenoxy]pentyl}-isoxazole [II; R₁ and R₂ = H, Het = 2-(1,3,4-oxadiazolyl)].

A mixture of 23.6 g 4-(1,3,4-oxadiazolyl) phenol (U.S. Pat. 4,218,458, Example XIX), 35 g 5-(5-bromopentyl)-3-methylisoxazole and 40 g milled potassium carbon to in 1.5 liters acetonitrile under nitrogen was heated to reflux. A catalytic amount of sodium iodide was added and refluxing continued for 4 hrs. The reaction mixture was filtered and concentrated to a solid residue. The latter was dissolved in ethyl acetate and the solution washed with water and saturated sodium chloride solution, dried over magnesium sulfate and concentrated in vacuo. The residue was recrystallized from triethylamine to give 18.5 g 3-methyl-5-{5-[4-(1,3,4-oxadiazol-2-yl)phenoxy]-pentyl} isoxazole, white needles, m.p. 84-86°C.

It is further contemplated that 4-(1,2,4-oxadiazol-5-yl)phenol can be caused to react with 5-(5-bromopentyl)-3-methylisoxazole according to the procedure of Example 1 to give 5-{5-[4-(1,2,4-oxadiazol-5-yl)phenoxy]-pentyl)-3-methylisoxazole [II; R₁ and R₂ = H, Het = 1,2,4-oxadiazol-5-yl].

### Example 2

a) 3,5-Dimethyl-4-[5-(3-methylisoxazol-5-yl)pentyloxy]-benzamide [XIV; Y = (CH₂)₅, R' = CH₃, R₁ and R₂ = 3,5-(CH₃)₂].
   A solution of 6.3 g 5-[5-(2,6-dimethyl-4-cyano-phenoxy)pentyl]-3-methylisoxazole [XIVa; Y = (CH₂)₅, R = CH₃, R₁ and R₂ = 2,6-(CH₃)₂; m.p. 50-51°C.] in 15 ml 97% sulfuric acid was stirred at room temperature for 1.5 hours. The reaction mixture was poured onto ice and extracted with ethyl acetate. The organic layer was washed with water, dried over sodium sulfate and concentrated in vacuo. The residue was recrystallized from isopropyl acetate-hexane and used directly in the next reaction.
b) 5-{5-[2,6-Dimethyl-4-(3-methyl-1,2,4-oxadiazol-5-yl)-phenoxy]pentyl}-3-methylisoxazole [XI; Y = (CH₂)₅, R′ = CH₃, R₁ and R₂ = 2,6-(CH₃)₂, R₈ = CH₃].
   A suspension of 2.7 g of the product of part (a) in 15 ml dimethylacetamide dimethyl acetal was heated at reflux under nitrogen for two hours. The reaction mixture was concentrated in vacuo and then treated with 0.7 g hydroxylamine hydrochloride, 2.0 ml 5N sodium hydroxide solution and 7.5 ml 70% aqueous acetic acid. The resulting mixture was stirred for 30 min. and then 4.5 ml water was added. The reaction mixture was extracted with ethyl acetate, and the organic layer was washed with water, dried over sodium sulfate and concentrated in vacuo. The residue was subjected to flash filtration (silica gel; 1:1 hexane/ethyl acetate), and chromatography (MPLC, silica gel 60; 3:1 hexane/ethyl acetate) to give 2.4 g (80%) of 5-{5-[2,6-dimethyl-4-(3-methyl-1,2,4-oxadiazol-5-yl)phenoxy]pentyl}-3-methylisoxazole, m.p. 54-56°C. when crystallized from isopropyl acetate/hexane.

### Example 3

a) 3,5-Dimethyl-4-[3-(3-methylisoxazol-5-yl) propyloxy]-benzamide [XIV; Y = (CH₂)₃, R′ = CH₃; R₁ and R₂ = 3,5-(CH₃)₂] was prepared from 6 g 5-[3-(2,6-dimethyl-4-cyanophenoxy)propyl]-3-methylisoxazole [XIVa; Y = (CH₂)₃, R = CH₃, R₁ and R₂ = 2,6-(CH₃)₂; m.p. 46-48°C. when recrystallized from methanol] according to the procedure of Example 2, part (a), and was obtained in 65% yield in the form of a colorless solid, m.p. 162-163°C. when recrystallized from methanol.
b) 5-{3-[2,6-Dimethyl-4-(3-methyl-1,2,4-oxadiazol-5-yl)-phenoxy]propyl}-3-methylisoxazole [XI; Y = (CH₂)₃, R′ = CH₃, R₁ and R₂ = 2,6-(CH₃)₂, R₈ = CH₃] was prepared from 6 g of the product of part (a), 30 g dimethylacetamide dimethyl acetal and 1.75 g hydroxylamine hydrochloride according to the procedure of Example 1, part (b), and was obtained (1.7 g) in the form of a colorless solid, m.p. 55.5-56.5°C. when recrystallized from isopropyl acetate-hexane.

It is contemplated that, according to the foregoing procedures, 5-[5-(2,6-dimethyl-4-cyanophenoxy)pentyl]-3-hydroxymethylisoxazole (yellowish-tan solid, m.p. 63-64°C. from diethyl ether) can be converted to 3,5-dimethyl-4-[5-(3-hydroxymethylisoxazol-5-yl)pentyloxy]benzamide [XIV; Y = (CH₂)₅, R′ = HOCH₂, R₁ and R₂ = 3,5-(CH₃)₂] and the latter converted to 5-{5-[2,6-dimethyl-4-(3-methyl-1,2,4-oxadiazol-5-yl)phenoxy]pentyl}-3-hydroxymethylisoxazole [XI; Y = (CH₂)₅, R′ = HOCH₂, R₁ and R₂ = 2,6-(CH₃)₂, R₈ = CH₃]. The intermediate nitrile was prepared from 2,6-dimethyl-4-cyanophenol and 5-(5-chloropentyl)-5-hydroxymethylisoxazole, the latter in turn prepared by reacting 1-bromo-4-chlorobutane with 3-hydroxymethyl-5-methylisoxazole in the presence of n-butyllithium. The 3-hydroxymethyl-5-methylisoxazole (b.p. 77-80°C., 0.5 mm) was in turn prepared by reduction of methyl 5-methylisoxazole-3-carboxylate with lithium aluminum hydride.

Similarly, it is contemplated that 5-[5-(2,6-dichloro-4-cyanophenoxy)pentyl]-3-methylisoxazole (m.p. 59-60°C.) can be converted to 5-{5-[2,6-dichloro-4-(3-methyl-1,2,4-oxadiazol-5-yl)phenoxy]pentyl}-3-methylisoxazole [XI; Y = (CH₂)₅, R′ = CH₃, R₁ and R₂ = 2,6-Cl₂, R₈ = CH₃].

### Example 4

a) 5-(3-Chloropropyl)-3-ethylisoxazole.
   A mixture of 11.56 g 1-nitropropane, 15 g 1-chloro-4-pentyne (HC≡C(CH₂)₃Cl], 37 g phenylisocyanate and 4 ml triethylamine in 300 ml toluene was stirred at room temperature for three days. The reaction mixture was filtered, the filtrate concentrated in vacuo, and the residue chromatographed (silica gel, eluted with hexane and 20% ethyl acetate in hexane) to give 12 g 5-(2-chloro-propyl)-3-ethylisoxazole as a yellow oil.
b) 5-[3-(2,6-Dimethyl-4-cyanophenoxy)propyl]-3-ethylisoxazole [XIVa; Y = (CH₂)₃, R′ = C₂H₅, R₁ and R₂ = 2,6-(CH₃)₂].
   To a mixture of 7.84 g 4-cyano-2,6-dimethylphenol, 3.57 g potassium hydroxide and 10.6 g potassium iodide in 200 ml acetonitrile was added 12 g 5-(2-chloro-propyl)-3-ethylisoxazole, and the mixture was heated at reflux for about 16 hrs. The reaction mixture was concentrated in vacuo and the residue partitioned between ethyl acetate and water. The ethyl acetate layer was washed with aqueous sodium hydroxide (10%) until the excess starting phenol was removed, and the solution concentrated to give 10 g of 5-[3-(2,6-dimethyl-4-cyanophenoxy)propyl]-3-ethylisoxazole as a yellow oil. A sample of the latter was obtained in crystalline form, m.p. 50-51°C. by crystallization from methanol at -50°C.
c) 3,5-Dimethyl-4-[3-(3-ethylisoxazol-5-yl)propyloxy]benzamide [XIV; Y = (CH₂)₃, R′ = C₂H₅, R₁ and R₂ = 3,5-(CH₃)₂] was prepared from the product of part (b) (10 g) by the procedure of Example 2, part (a), and was obtained (8.7 g) as a solid, m.p. 132-134°C. when washed with ethyl acetate.
d) 5-{3-[2,6-Dimethyl-4-(3-methyl-1,2,4-oxadiazol-5-yl)-phenoxy]propyl}-3-ethylisoxazole [XI; Y = (CH₂)₃, R′ = C₂H₅, R₁ and R₂ = 2,6-(CH₃)₂, R₈ = CH₃] was prepared from 4.15 g of the product of part (c), 20.5 g dimethylacetamide dimethyl acetal and 1.2 g hydroxylamine hydrochloride according to the procedure of Example 1, part (b), and was obtained (2.1 g) in the form of a colorless solid, m.p. 40-41°C. when recrystallized from isopropyl acetate/hexane.

### Example 5

a) Ethyl 3,5-dimethyl-4-(3-ethynylpropoxy) benzoate.
   A suspension of 6.42 g ethyl 3,5-dimethyl-4-hydroxybenzoate, 5.5 g potassium iodide, 3.7 g 1-chloro-4-pentyne and 2.3 g powdered potassium hydroxide in 650 ml acetonitrile was heated at reflux under nitrogen for 16 hrs. The mixture was filtered, concentrated in vacuo and subjected to flash filtration (silica gel, 4:1 hexane/ethyl acetate) to give 7.4 g ethyl 3,5-dimethyl-4-(3-ethynylpropoxy)benzoate as a yellow oil.
b) 3,5-Dimethyl-4-(3-ethynylpropoxy)benzoic acid.
   The ethyl ester of part (a) (7.4 g) was hydrolyzed, first with 0.9 g lithium hydroxide in 25 ml methanol and 10 ml water, stirred at room temperature for 3 days, and then with 50 ml 10% sodium hydroxide in 100 ml ethanol, heated at reflux for 2 hrs. The product was isolated by acidification, extraction with ether and concentration to give 4.7 g 3,5-dimethyl-4-(3-ethynylpropoxy)-benzoic acid, m.p. 129-130°C.
c) 3,5-Dimethyl-4-(3-ethynylpropoxy)benzamide.
   To a solution of 4.7 g 3,5-dimethyl-4-(3-ethynylpropoxy)benzoic acid in 100 ml dry tetrahydrofuran was added 3.7 g carbonyldiimidazole and the mixture was stirred 2 hrs. at room temperature. To the latter was added 10 ml concentrated ammonium hydroxide, and the mixture was stirred for 30 min., poured onto water and extracted with ethyl acetate. The organic layer was dried (MgSO₄), filtered and concentrated to give 4.7 g 3,5-dimethyl-4-(3-ethynylpropoxy)benzamide, m.p. 81-84°C.
d) 5-[3,5-Dimethyl-4-(3-ethynylpropoxy)phenyl]-3-methyl-1,2,4-oxadiazole [XV; Y = (CH₂)₃, R₁ and R₂ = 3,5-(CH₃)₂, R₈ = CH₃] was prepared from 4.7 g 3,5-dimethyl-4-(3-ethynylpropoxy)benzamide, 31 ml dimethylacetamide dimethyl acetal and 1.7 g hydroxylamine hydrochloride according to the procedure of Example 2, part (b), and was obtained (4.4 g) in the form of a viscous oil used directly in the next reaction. A sample when crystallized from isopropyl acetate/hexane had the m.p. 38-40°C.
e) 5-{3-[2,6-Dimethyl-4-(3-methyl-1,2,4-oxadiazol-5-yl)-phenoxy]propyl}-3-(n-propyl)isoxazole [XI; Y = (CH₂)₃, R′ = CH₃CH₂CH₂, R₁ and R₂ = 2,6-(CH₃)₂, R₈ = CH₃].
   To a solution of 4.4 g of the product of part (d), 1.7 g 1-nitrobutane and 12 drops triethylamine in 40 ml dry toluene was added a solution of 3.7 ml phenylisocyanate in 5 ml dry toluene dropwise over a 2 hr. period. The reaction mixture was stirred at room temperature for five days under nitrogen, then filtered, concentrated and subjected to chromatography (MPLC, silica gel, 3:1 hexane/ethyl acetate). A first fraction brought out 1.2 g of starting material of part (d), and a second fraction 3.0 g of the desired product which was recrystallized from isopropyl acetate/hexane at -50°C. to give 2.5 g 5-{3-[2,6-dimethyl-4-(3-methyl-1,2,4-oxadiazol-5-yl)phenoxy]propyl}-3-(n-propyl)isoxazole, m.p. 35-37°C.

### Example 6

### 5-{5-[2,6-Dimethyl-4-(5-methyl-1,2,4-oxadiazol-3-yl)phenoxy]pentyl}-3-methylisoxale [XII; Y = (CH₂)₅, R′ = CH₃, R₁ and R₂ = 2,6-(CH₃)₂, R₈ = CH₃].

A suspension of 7.0 g 5-[5-(2,6-dimethyl-4-cyanophenoxy)pentyl]-3-methylisoxazole, 1.8 g hydroxylamine hydrochloride and 3.5 g sodium acetate trihydrate in 40 ml 95% ethanol was heated at reflux under nitrogen for two days. The reaction mixture was filtered, concentrated in vacuo, and the residue in 50 ml acetic anhydride was heated at reflux for 3 hrs. The reaction mixture was poured over ice/10% sodium hydroxide and extracted with ether. The ether extracts were washed with water and saturated sodium bicarbonate, dried (K₂CO₃), concentrated and subjected to flash filtration (silica gel 60, 3:1 hexane/ethyl acetate) followed by chromatography (MPLC, silica gel 60, 3:1 hexane/ethyl acetate). The resulting product was crystallized from isopropyl acetate/hexane to give 2.7 g 5-{5-[2,6-dimethyl-4-(5-methyl-1,2,4-oxadiazol-3-yl)phenoxy]pentyl}-3-methylisoxazole, m.p. 45-46°C.

Similarly, it is contemplated that 5-[5-(2,6-dimethyl-4-cyanophenoxy)pentyl]-3-hydroxymethylisoxazole or 5-[5-(2,6-dichloro-4-cyanophenoxy)pentyl]-3-methylisoxazole can be converted, respectively, to 5-{5-[2,6-dimethyl-4-(5-methyl-1,2,4-oxadiazol-3-yl)phenoxy] pentyl}-3-hydroxymethylisoxazole [XII; Y = (CH₂)₅, R′ = HOCH₂, R₁ and R₂ = 2,6-(CH₃)₂, R₈ = CH₃], or 5-{5-[2,6-dichloro-4-(5-methyl-1,2,4-oxadiazol-3-yl)phenoxy] pentyl}-3-methylisoxazole [XII; Y = (CH₂)₅, R′ = CH₃, R₁ and R₂ = 2,6-Cl₂, R₈ = CH₃].

### Example 7

5-{3-[2,6-Dimethyl-4-(5-methyl-1,2,4-oxadiazol-3-yl)phenoxy]propyl}-3-methylisoxasole [XII; Y = (CH₂)₃, R′ = CH₃, R₁ and R₂ = 2,6-(CH₃)₂, R₈ = CH₃] was prepared from 6 g of the imino chloride derived by reaction of 5-[3-(2,6-dimethyl-4-cyanophenoxy)propyl]-3-methylisoxazole with ethanolic hydrogen chloride, 1.49 g of hydroxylamine hydrochloride and 100 ml acetic anhydride according to the procedure of Example 6, and was obtained (4 g) as a colorless solid, m.p. 80-82°C. when recrystallized from methanol.

### Example 8

a) 3,5-Dimethyl-4-(3-ethynylpropoxy)benzonitrile.
   A mixture of 40 g 3,5-dimethyl-4-hydroxybenzonitrile, 30.77 g 1-chloro-4-pentyne, 74.63 g potassium carbonate and 130 ml N-methylpyrrolidinone was heated with stirring to 100°C. and stirring was continued at 100°C. for 4 hrs. The mixture was cooled to room temperature and 800 ml of water was added. This mixture was extracted with 400 ml ethyl acetate and the extract was washed with 2N sodium hydroxide and saturated sodium chloride solution, dried over anhydrous magnesium sulfate and concentrated in vacuo. The resulting black oil, dissolved in ethyl acetate, was passed through a pad of florisil to remove the color and the filtrate was concentrated in vacuo. The residue was recrystallized from methyl alcohol to yield 34.64 g (60.2%) of 3,5-dimethyl-4-(3-ethynyl-propoxy) benzonitrile, m.p. 48-49°C.
b) 3-[3,5-Dimethyl-4-(3-ethynylpropoxy)phenyl]-5-methyl-1,2,4-oxadiazole.
   A mixture of 25 g of the benzonitrile of part (a), 16.6 g of hydroxylamine hydrochloride, 32.64 g sodium acetate trihydrate, 200 ml ethyl alcohol and 40 ml water was refluxed for 5 hrs and cooled to room temperature. The ethyl alcohol was distilled off in vacuo and the residue, after standing overnight at room temperature, was suspended in 200 ml acetic anhydride and the mixture was refluxed for 2 hrs and allowed to cool to room temperature. Sodium hydroxide (2N) was added slowly with stirring and ice bath cooling during which time heat and gas were evolved. The mixture was extracted with ether and the extract was washed successively with dilute sodium hydroxide solution, dilute sodium bicarbonate solution and saturated salt solution. The extract was dried over anhydrous sodium sulfate and concentrated in vacuo to a brown oil. A solution of the oil in ethyl acetate was passed through a column of florisil to remove color and concentrated in vacuo. The residue was subjected to chromatography (MPLC, silica gel, 15:85 ethyl acetate/hexane) to give 17.8 g (56%) of 3-[3,5-dimethyl-4-(3-ethynylpropoxy) phenyl]-5-methyl-1,2,4-oxadiazole as a white solid, m.p. 41.5-43.0°C.
c) 5-{3-[2,6-Dimethyl-4-(5-methyl-1,2,4-oxadiazol-3-yl)-phenoxy]propyl}-3-ethylisoxazole [XII; Y = (CH₂)₃, R′ = CH₃CH₂, R₁ and R₂ = 2,6-(CH₃)₂, R₈ = CH₃].
   A mixture of 3.99 g N-chlorosuccinimide, 2 drops pyridine and 75 ml N-methypyrrolidinone was stirred at room temperature until clear and 2.19 g propionaldehyde oxime was added over 10 minutes. Stirring was continued for ½ hour at room temperature and 2.7 g of the 1,2,4-oxadiazole of part (b) in 15 ml N-methylpyrrolidinone was added in one portion. The mixture was heated to 90°C., 4.2 ml triethylamine were added over 3 hrs and stirring and heating were continued for 1 hr. The mixture was cooled to room temperature, suspended in water and extracted with methylene chloride. The extract was washed with water and saturated salt solution, dried over anhydrous magnesium sulfate and concentrated in vacuo. The residue was subjected to chromatography (MPLC, silica gel, 35:65 ethyl acetate/hexane) to give 1.86 g (55%) of 5-{3-[2,6-dimethyl-4-(5-methyl-1,2,4-oxadiazol-3-yl)phenoxy]propyl}-3-ethylisoxazole as a white solid, m.p. 85-86°C. after recrystallization from methyl alcohol.

### Example 9

### 5-{3-[2,6-Dimethyl-4-(5-methyl-1,2,4-oxadiazol-3-yl)phenoxy]propyl}-3-propylisoxazole [XII: Y (CH₂)₃, R′ = CH₃CH₂CH₂, R₁ and R₂ = 2,6-(CH₃)₂, R₈ = CH₃].

Following a procedure similar to that of Example 8(c) but substituting 2.6 g butyraldehyde oxime for the propionaldehyde oxime and using 3.99 g N-chlorosuccinimide, 2 drops pyridine, 50 ml N-methylpyrrolidinone, 2.7 g of the 1,2,4-oxadiazole of part (b) of Example 8 and 4.2 ml triethylamine, there was obtained after chromatography (MPLC, silica gel, 30:70 ethyl acetate/hexane) 2.06 g (58%) of the title compound as white needles, m.p. 69.0-70.0°C. when recrystallized from methyl alcohol.

### Example 10 (Comparative)

a) 3,5-Dichloro-4-hydroxybenzoic acid hydrazide.
   A mixture of 10.0 g methyl 3,5-dichloro-4-hydroxybenzoate and 15 ml hydrazine hydrate was warmed on a steam bath for 3 hrs. Excess hydrazine was removed in vacuo and the residue recrystallized from 2-propinolwater (80:20) to give 9 g of the hydrazide, used directly in the next reaction.
b) 2,6-Dichloro-4-(1,3,4-oxadiazol-2-yl)phenol [IV; R₁ = 2-Cl, R₂ = 6-Cl, Het = 1,3,4-oxadiazol-2-yl].
   A mixture of 8.9 g 3,5-dichloro-4-hydroxybenzoic acid hydrazide and 500 ml triethyl orthoformate was stirred and heated at reflux for 4 hrs. The solvent was removed in vacuo to afford the product (9.9 g) as a yellow solid, used directly in the next reaction.
c) 5-{5-[2,6-Dichloro-4-(1,3,4-oxadiazol-2-yl)phenoxy]-pentyl}-3-methylisoxazole [II; R₁ and R₂ = Cl, Het = 1,3,4-oxadiazol-2-yl] was prepared from 8.5 g 2,6-dichloro-4-(1,3,4-oxadiazol-2-yl)phenol and 20 g 5-(5-bromopentyl)-3-methylisoxazole according to the procedure of Example 1, and was obtained in 34% yield (4.8 g , m.p. 73-74°C. when recrystallized from triethylamine.

It is further contemplated that by substituting for the 5-(5-bromopentyl)-3-methylisoxazole in the foregoing procedure a molar equivalent amount of 5-(5-chloropentyl)-3-hydroxymethylisoxazole there can be prepared 5-{5-[2,6-dichloro-4-(1,3,4-oxadiazol-2-yl)phenoxy]pentyl}-3-hydroxymethylisoxazole [XIII; R' = HOCH₂, R₁ and R₂ = 2,6-Cl₂, Y = (CH₂)₅]. The intermediate 5-(5-chloropentyl)-3-hydroxymethylisoxazole was prepared by reacting 1-bromo-4-chlorobutane with 3-hydroxymethyl-5-methylisoxazole in the presence of n-butyllithium. The 3-hydroxy-methyl-5-methylisoxazole (b.p. 77-80°C., 0.5 mm) was in turn prepared by reduction of methyl 5-methylisoxazole-3-carboxylate with lithium aluminum hydride.

### Example 11

a) 3,5-Dichloro-4-[5-(3-methylisoxazol-5-yl)pentyloxyl-benzoic acid [XVIII; R′ = CH₃, Y = (CH₂)₅, R₁ and R₂ = 3,5-Cl₂].
   A mixture of 6.11 g 5-[5-(2,6-dichloro-4-cyanophenoxy)pentyl]-3-methylisoxazole (m.p. 59-60°C.), 75 ml 35% sodium hydroxide and 75 ml ethanol was stirred on a steam bath for several hours. The reaction mixture upon standing separated into a clear lower layer and a yellow upper layer. The latter was separated, diluted with cold water and acidified with hydrochloric acid. The resulting solid was collected and recrystallized from acetonitrile to give 5.24 g 3,5-dichloro-4-[5-(3-methylisoxazol-5-yl)-pentyloxy]benzoic acid, m.p. 67-69°C.
b) N-Acetyl-N′-{3,5-dichloro-4-[5-(3-methylisoxazol-5-yl)-pentyloxy]benzoyl}hydrazine [XVII; R′ = CH₃, Y = (CH₂)₅, R₁ and R₂ = 3,5-Cl₂, R₈ = CH₃].
   A solution of 5.19 g of the product of part (a) and 2.59 g carbonyldiimidazole in 50 ml tetrahydrofuran (THF) in a nitrogen atmosphere was heated at reflux until gas evolution ceased. The mixture was cooled and 1.18 g acetylhydrazine added. The resulting mixture was heated at reflux for 5-6 hours and then concentrated in vacuo. The residue was dissolved in ethyl acetate and filtered through a plug of silica gel. There was isolated a colorless solid (1.49 g), N-acetyl-N′-{3,5-dichloro-4-[5-(3-methylisoxazol-5-yl)pentyloxy]benzoyl}hydrazine, m.p. (polymorphic) 118° and 134°C. when recrystallized from isopropyl acetate.
c) 5-{5-[2,6-Dichloro-4-(5-methyl-1,3,4-oxadiazol-2-yl)-phenoxy]pentyl}-3-methylisoxazole [XIII; R′ = CH₃, Y = (CH₂)₅, R₁ and R₂ = 2,6-Cl₂, R₈ = CH₃].
   A mixture of 0.94 g of the product of part (b), 1 ml hexamethyldisilazane, 0.5 ml tetrabutylammonium fluoride and 10 ml chlorobenzene was heated to 125°C. under nitrogen with stirring. A catalytic amount of imidazole as a silylating catalyst was then added. After one-half hour an additional 0.5 ml hexamethyldisilazane and a catalytic amount of tetrabutylammonium fluoride were added and the reaction mixture heated at 125°C. for three days. The mixture was then cooled and filtered through a plug of silica gel and eluted with ethyl acetate. The product isolated from the filtrate was recrystallized from isopropyl acetate-hexane to give 0.53 g 5-{5-[2,6-dichloro-4-(5-methyl-1,3,4-oxadiazol-2-yl)phenoxy]pentyl}-3-methylisoxazole, light tan solid, m.p. 71-72°C.

It is further contemplated that by replacing the 5-[5-(2,6-dichloro-4-cyanophenoxy)pentyl]-3-methylisoxazole in part (a) by a molar equivalent amount of 5-[5-(2,6-dimethyl-4-cyanophenoxy)pentyl]-3-hydroxymethylisoxazole there can be prepared 3,5-dimethyl-4-[5-(3-hydroxymethylisoxazol-5-yl)pentyloxy]benzoic acid [XVIII R′ = HOCH₂, Y = (CH₂)₅, R₁ and R₂ = 3,5-(CH₃)₂], N-acetyl-N′-{3,5-dimethyl-4-[5-(3-hydroxymethylisoxazol-5-yl)pentyloxy]benzoyl}hydrazine [XVII; R′ = HOCH₂, Y = (CH₂)₅, R₁ and R₂ = 3,5-(CH₃)₂, R₈ = CH₃] and 5-{5-[2,6-dimethyl-4-(5-methyl-1,3,4-oxadiazol-2-yl)phenoxy] pentyl}-3-hydroxymethylisoxazole [XIII; R′ = HOCH₂, Y = (CH₂)₅, R₁ and R₂ = 2,6-(CH₃)₂, R₈ = CH₃]. The intermediate 5-[5-(2,6-dimethyl-4-cyanophenoxy)pentyl]-3-hydroxymethyl-isoxazole (yellowish-tan solid, m.p. 63-64°C. from diethyl ether) was prepared from 2,6-dimethyl-4-cyanophenol and 5-(5-chloropentyl)-3-hydroxymethylisoxazole.

### Example 12

a) Ethyl 3,5-dimethyl-4-[3-(3-methylisoxazol-5-yl)propyloxy]benzoate.
   To a mixture of 10.05 g ethyl 3,5-dimethyl-4-hydroxybenzoate and 8.0 g 5-(3-chloropropyl)-3-methylisoxazole in 550 ml dry acetonitrile was added 4.87 g potassium hydroxide and 12.9 g potassium iodide. The reaction mixture was stirred at reflux overnight and then filtered and concentrated in vacuo. The residue was partitioned between ethyl acetate and water and the organic layer concentrated to a yellow oil. The latter was crystallized from ethyl acetate-hexane (1:4) at -50°C. to give 14 g of product, 12 g of which was used directly in the following hydrolysis reaction. A sample of the ester was purified by chromatography on silica to give pure compound, m.p. 49-50°C.
b) 3,5-Dimethyl-4-[3-(3-methylisoxazol-5-yl) propyloxyl-benzoic acid [XVIII; R′ = CH₃, Y = (CH₂)₃, R₁ and R₂ = 3,5-(CH₃)₂] was prepared by hydrolysis of 12 g of the crude product from part (a) with 120 ml 10% sodium hydroxide in 120 ml ethanol, stirred at reflux for two hours. The reaction mixture was acidified and the product isolated to give 9 g 3,5-dimethyl-4-[3-(3-methylisoxazol-5-yl)-propyloxy]benzoic acid, m.p. 156-157°C. when recrystallized from isopropyl acetate-hexane (1:1).
c) N-Acetyl-N′-{3,5-dimethyl-4-[3-(3-methylisoxazol-5-yl)-propyloxy]benzoyl}hydrazine [XVII; R′ = CH₃, Y = (CH₂)₃, R₁ and R₂ = 3,5-(CH₃)₂, R₈ = CH₃] was prepared from 8.3 g of the product of part (b) according to the procedure of Example 11, part (b), and was obtained (10.2 g) in the form of a colorless solid, m.p. 141-142°C. when recrystallized from ethyl acetate-hexane (1:1).
d) 5-{3-[2,6-Dimethyl-4-(5-methyl-1,3,4-oxadiazol-2-yl)-phenoxy]propyl}-3-methylisoxazole [XIII; R′ = CH₃, Y = (CH₂)₃, R₁ and R₂ = 2,6-(CH₃)₂, R₈ = CH₃] was prepared from 10 g of the product of part (c) according to the procedure of Example 11, part (c), and was obtained (0.6 g after chromatography on silica) in the form of a colorless solid, m.p. 99-100°C. when recrystallized from isopropyl acetate.

### Example 13 3-Methyl-5-{7-[4-(5-methyl-1,3,4-oxadiazol-2-

### yl)phenoxy]-heptyl}isoxazole [XIII; R′ = CH₃, Y = (CH₂)₇, R₁ and R₂ = H, R₈ = CH₃].

A mixture of 1.3 g 5-{7-[4-(1H-tetrazol-5-yl)-phenoxy]heptyl}-3-methylisoxazole [U.S. Patent 4,857,539, Example 48(a)], 50 mg hydroquinone and 10 ml acetic anhydride under nitrogen was stirred and heated at reflux for one hour, allowed to stand at room temperature overnight, treated with cold water and stirred for about three hours. The mixture was filtered and the resulting tan solid was recrystallized from methyl alcohol to give 1.09 g (81%) of the title compound, m.p. 93-95°C.

### Example 14

### 5-{5-[2,6-Dimethyl-4-(5-methyl-1,3,4-oxadiazol-2-yl)phenoxy]pentyl}-3-methylisoxazole [XIII; R′ = CH₃, Y = (CH₂)₅, R₁ and R₂ = 2,6-(CH₃)₂, R₈ = CH₃].

A mixture of 2.39 g 5-(5-[2,6-dimethyl-4-(1H-tetrazol-5-yl)phenoxy]pentyl}-3-methylisoxazole [U.S. Patent 4,857,539, Example 49(a)], 100 mg hydroquinone and 20 ml acetic anhydride under nitrogen was stirred and heated at reflux for one hour, allowed to stand at room temperature overnight and poured into 150 ml cold water with stirring. The aqueous mixture was extracted with ethyl acetate and the extract was washed successively with water, saturated sodium bicarbonate solution, water and saturated sodium chloride solution, dried over magnesium sulfate, and concentrated in vacuo to give, when recrystallized from isopropyl acetate, 1.2 g (48%) of the title compound, m.p. 74-75°C.

Biological evaluation of compounds of Formulas I and II has shown that they possess antiviral activity. They are useful in inhibiting virus replication in vitro and are primarily active against picornaviruses, including enteroviruses, polioviruses, echovirus and coxsackie virus, and especially numerous strains of rhinoviruses. The in vitro testing of the compounds of the invention against picoraviruses showed that viral replication was inhibited at minimum inhibitory concentrations (MIC) ranging from about 0.01 to about 5 micrograms per milliliter.

The MIC values were determined by a standard plaque reduction assay as follows: HeLa (Wisconsin) cells in monolayers were infected at a concentration of virus to give approximately 80 plaques per monolayer in the virus control (no drug present). The compound to be tested was serially diluted and included in the agar-medium overlay and in some cases, during the adsorption period as well. The MIC was determined to be that concentration of compound which reduced the number of plaques by 50% with respect to the untreated virus control.

In the standard test procedures, the compounds were tested against a panel of fifteen human rhinovirus (HRV) serotypes, namely HRV-2, -1A, -1B, -6, -14, -21, -22, -15, -25, -30, -50, -67, -89, -86 and -41. The MIC value for each rhinovirus serotype was determined, and the efficacy of each compound was determined in terms of MIC₅₀ and MIC₈₀ values, which is the concentration of the compound required to inhibit 50% and 80%, respectively, of the tested serotypes.

The following Table gives the testing results with the compounds of the invention. For some of the compounds, the MIC₅₀ and MIC₈₀ values are based on the testing of fewer than 15 rhinovirus serotypes. In these cases the number of serotypes (N) is indicated in parentheses after the MIC₈₀ figure.

| Example No. | MIC (Polio 2) | MIC₅₀ (Rhinovirus) | MIC₈₀ (N) (Rhinovirus) |
|---|---|---|---|
| 1 (Comparative) | 0.05 | | 2.56 (1) |
| 2(b) | | 0.053 | 0.42 (6) |
| 3(b) | | 0.026 | 0.12 (15) |
| 4(d) | | 0.042 | 0.15 (15) |
| 5(e) | | 0.036 | 0.06 (7) |
| 6 | | 0.018 | 0.10 (5) |
| 7 | | 0.012 | 0.069 (13) |
| 8(c) | | 0.017 | 0.05 (6) |
| 9 | | 0.025 | 0.04 (6) |
| 10(c) (Comparative) | 2.24 | 0.25 | 0.91 |
| 11(c) | | 0.14 | 0.46 (15) |
| 12(d) | | 0.054 | 0.35 (6) |
| 13 | | 2.3 | 3.2 (2) |
| 14 | | 0.34 | 0.67 (2) |

The following Table compares the MIC values for the compound of Example 10(c) and the methylated derivative of Example 11(c) against 15 rhinovirus serotypes:

| Serotype | Example 10(c) (Comparative) | Example 11(c) |
|---|---|---|
| 1A | 1.45 | 0.44 |
| 1B | 0.53 | 0.14 |
| 2 | 0.41 | 0.016 |
| 6 | 7.06 | 0.46 |
| 14 | 2.02 | 0.19 |
| 15 | 1.92 | 0.42 |
| 21 | 0.24 | 0.0092 |
| 22 | 0.45 | 0.018 |
| 25 | 2.42 | 0.7 |
| 30 | 0.10 | 0.031 |
| 41 | 2.76 | 99* |
| 50 | 0.091 | 0.041 |
| 67 | 0.64 | 0.1 |
| 86 | 1.28 | 0.57 |
| 89 | 0.28 | 0.01 |

| | | |
|---|---|---|
| *Inactive at dose levels tested | | |

The foregoing data show that, except in the case of serotype 41, the compound of Example 11(c) is substantially more active than the compound of Example 10(c).

The antiviral compositions are formulated for use by preparing a dilute solution or suspension in a pharmaceutically acceptable aqueous, organic or aqueous-organic medium for topical or parenteral administration by intravenous or intramuscular injection, or for intranasal or ophthalmic application; or are prepared in tablet, capsule, or aqueous suspension form with conventional excipients for oral administration.

## Claims (Claims for the following Contracting State(s): DK, DE, GB, FR, IT, NL, SE, LI, CH, BE, AT)

1. A compound of the formula 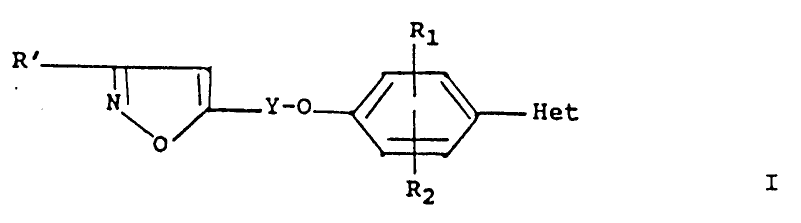 wherein:
Het is selected from 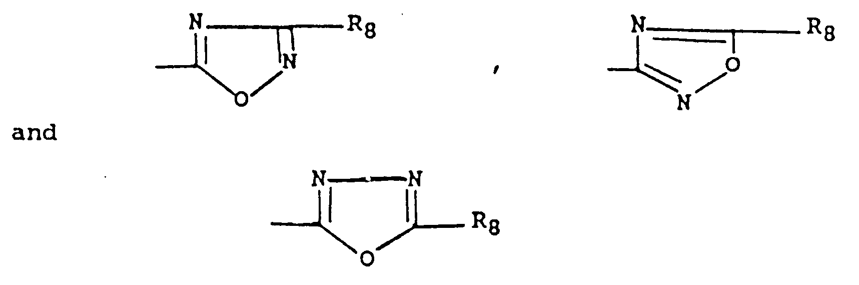 Y is an alkylene bridge of 3-9 carbon atoms;
R' is C₁₋₅ alkyl or hydroxy-C₁₋₅ alkyl;
R₁ and R₂ are hydrogen, halogen, C₁₋₅ alkyl, C₁₋₅ alkoxy, nitro, C₂₋₄-alkoxycarbonyl or trifluoromethyl; and
R₈ is alkyl of 1-5 carbon atoms;
or pharmaceutically acceptable acid-addition salts thereof.

2. A compound according to claim 1, of the formula 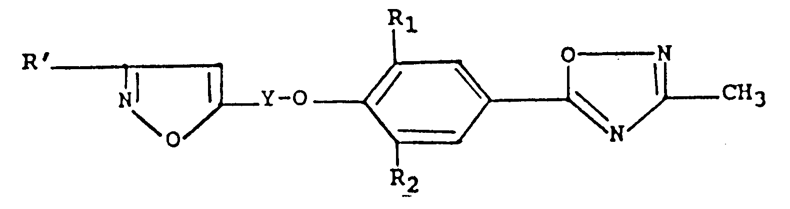 wherein Y is an alkylene bridge of 3-9 carbon atoms; R' is alkyl of 1-5 carbon atoms; and R₁ and R₂ are hydrogen, halogen or C₁₋₅-alkyl.

3. A compound according to claim 2, selected from 5-{5-[2,6-dimethyl-4-(3-methyl-1,2,4-oxadiazol-5-yl)phenoxy] pentyl}-3-methylisoxazole, 5-{3-[2,6-dimethyl-4-(3-methyl-1,2,4-oxadiazol-5-yl)phenoxy]propyl}-3-methylisoxazole, 5-{3-[2,6-dimethyl-4-(3-methyl-1,2,4-oxadiazol-5-yl)phenoxy] propyl}-3-ethylisoxazole, and 5-{3-[2,6-dimethyl-4-(3-methyl-1,2,4-oxadiazol-5-yl)phenoxy]propyl}-3-propylisoxazole.

4. A compound according to claim 1, of the formula 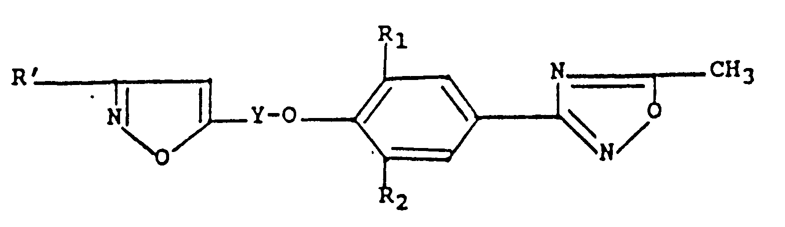 wherein Y is an alkylene bridge of 3-9 carbon atoms; R' is alkyl of 1-5 carbon atoms; and R₁ and R₂ are hydrogen, halogen or C₁₋₅ alkyl.

5. A compound according to claim 4, selected from 5-{5-[2,6-dimethyl-4-(5-methyl-1,2,4-oxadiazol-3-yl)phenoxy] pentyl}-3-methylisoxazole, 5-{3-[2,6-dimethyl-4-(5-methyl-1,2,4-oxadiazol-3-yl)phenoxy]propyl}-3-methylisoxazole, and 5-{3-[2,6-dimethyl-4-(5-methyl-1,2,4-oxadiazol-3-yl)phenoxy] propyl}-3-ethylisoxazole.

6. A compound according to claim 1, of the formula 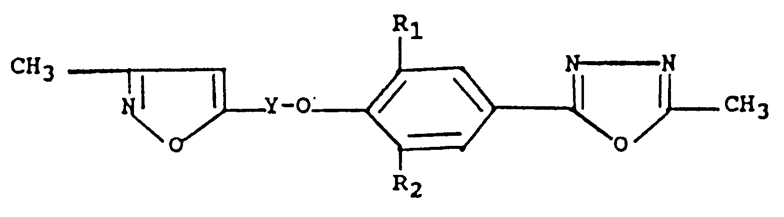 wherein Y, R₁ and R₂ have the meanings given in claim 1.

7. A compound according to claim 6, selected from 5-{5-[2,6-dichloro-4-(5-methyl-1,3,4-oxadiazol-2-yl)phenoxy] pentyl}-3-methylisoxazole, and 5-{3-[2,6-dichloro-4-(5-methyl-1,3,4-oxadiazol-2-yl)phenoxy]propyl}-3-methylisoxazole.

8. A composition for combating picornaviruses which comprises an antivirally effective amount of a compound according to any one of the preceding claims, in admixture with a suitable carrier or diluent.

9. A composition according to claim 8, for combating rhinoviruses.

10. A compound of the formula 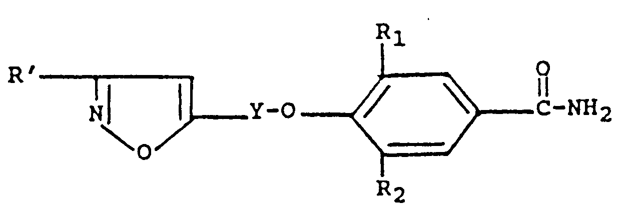 wherein Y is an alkylene bridge of 3-9 carbon atoms; R' is C₁₋₅ alkyl or hydroxy-alkyl of 1-5 carbon atoms; and R₁ and R₂ are halogen or C₁₋₅-alkyl.

11. A compound according to claim 10, selected from 3,5-dimethyl-4-[3-(3-methylisoxazol-5-yl)propyloxy] benzamide, and 3,5-dimethyl-4-[3-(3-ethylisoxazol-5-yl)propyloxy]benzamide.

12. A process for preparing a compound according to claim 1, which comprises reacting a compound of the formula 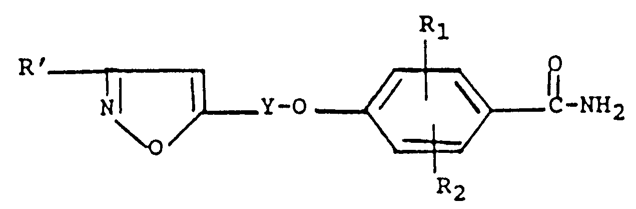 with a compound of the formula (CH₃)₂N-C(OCH₃)₂-R₈, followed by reaction of the resulting product with hydroxylamine in acetic acid solution to produce a compound of the formula

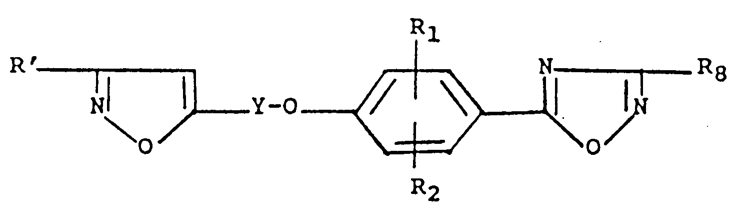

13. A process for preparing a compound according to claim 1, which comprises reacting a compound of the formula 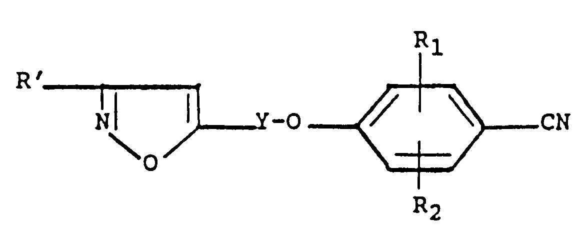 or the imino chloride derivative thereof with hydroxylamine, followed by reaction of the resulting product with an anhydride of the formula (R₈CO)₂O to produce a compound of the formula

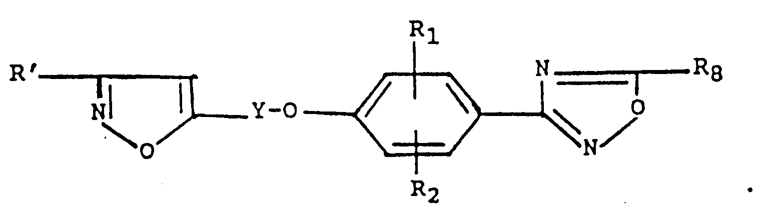

14. A process for preparing a compound according to claim 1, which comprises reacting a compound of the formula 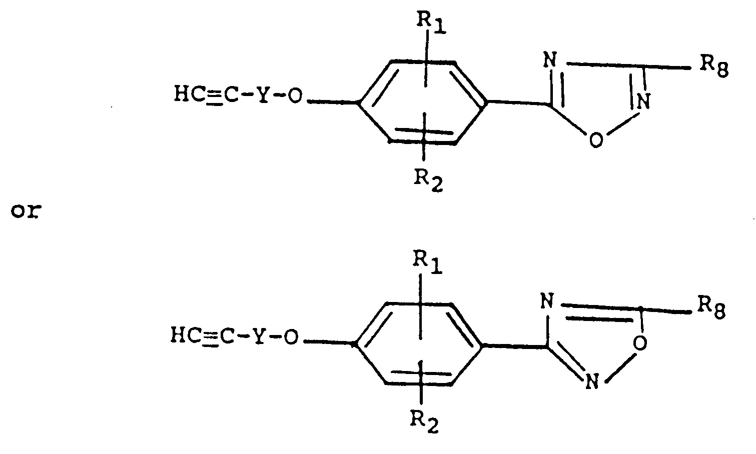 with a compound of the formula R'CH₂NO₂ and phenylisocyanate.

15. A process for preparing a compound according to claim 1, which comprises reacting a compound of the formula 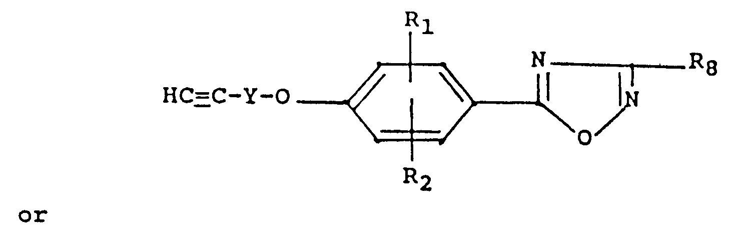 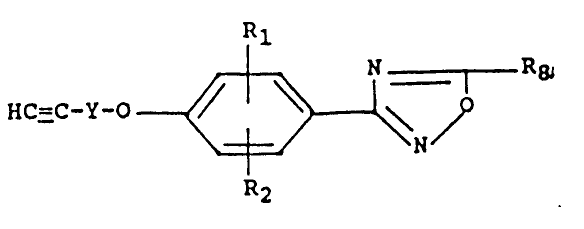 with a compound of the formula R'CH=NOH and N-halosuccinimide where halo is bromo or chloro.

16. A process for preparing a compound according to claim 1, which comprises reacting a compound of the formula 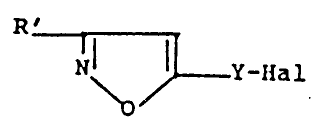 with a compound of the formula 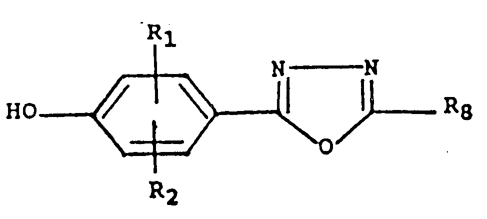 where Hal is chlorine, bromine or iodine.

17. A process for preparing a compound according to claim 1, which comprises reacting a compound of the formula 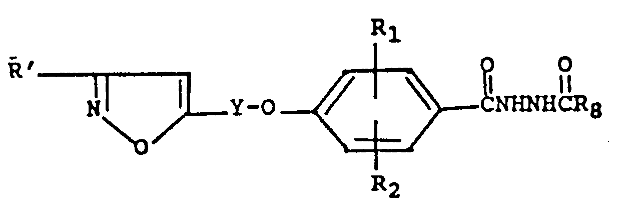 with hexamethyldisilazane in the presence of tetrabutylammonium trifluoride.

18. A process for preparing a compound according to claim 1, where R' is alkyl of 1-5 carbon atoms and Het is 5-C₁₋₅alkyl-1,3,4-oxadiazolyl which comprises reacting a compound of the formula 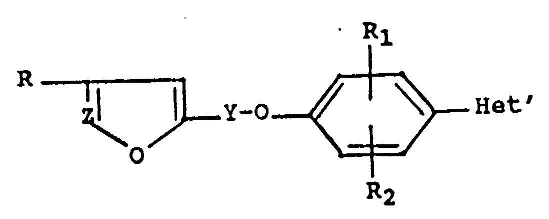 wherein:
Y is an alkylene bridge of 3-9 carbon atoms;
Z is N;
R is alkyl of 1-5 carbon atoms;
R₁ and R₂ are hydrogen, halogen, C₁₋₅-alkyl, C₁₋₅ alkoxy, nitro, C₂₋₄-alkoxycarbonyl or trifluoromethyl; and
Het' is 1H-tetrazol-5-yl
with an alkanoic acid chloride of the formula R₈CO-Hal'' where Hal'' is chlorine or bromine, or an alkanoic acid anhydride of the formula (R₈CO)₂O.

19. Compound according to any one of claims 1-7 for use in controlling viral infections.

20. Use of a compound according to any one of claims 1-7 as a non therapeutic antiviral agent.

21. Use of a compound according to any one of claims 1-7 for preparing a medicament to combat viral infections.

## Claims (Claims for the following Contracting State(s): ES)

1. A process for preparing compound of the formula 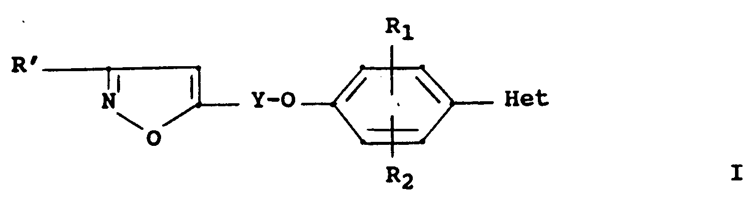 wherein:
Het is selected from 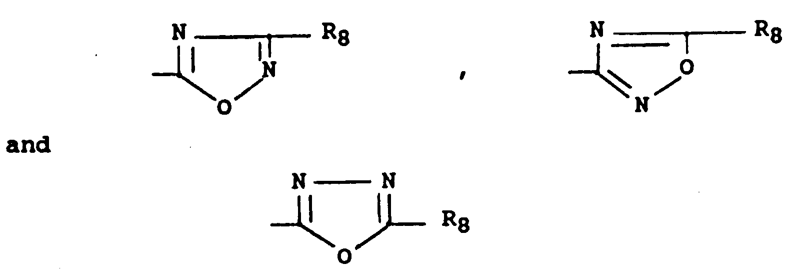 Y is an alkylene bridge of 3-9 carbon atoms;
R' is C₁₋₅ alkyl or hydroxy-C₁₋₅ alkyl atoms;
R₁ and R₂ are hydrogen, halogen, C₁₋₅ alkyl, C₁₋₅ alkoxy, nitro, C₁₋₅ alkoxycarbonyl or trifluoromethyl; and
R₈ is alkyl of 1-5 carbon atoms; or pharmaceutically acceptable acid-addition salts thereof
by one of the following reactions:
i) reacting a compound of the formula 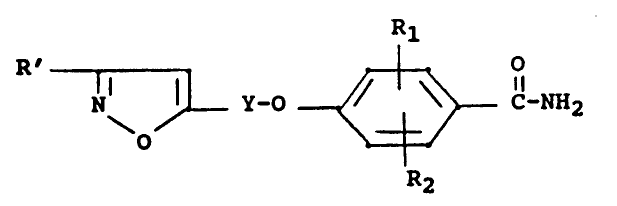 with a compound of the formula (CH₃)₂N-C(OCH₃)₂-R₈, followed by reaction of the resulting product with hydroxylamine in acetic acid solution to produce a compound of the formula 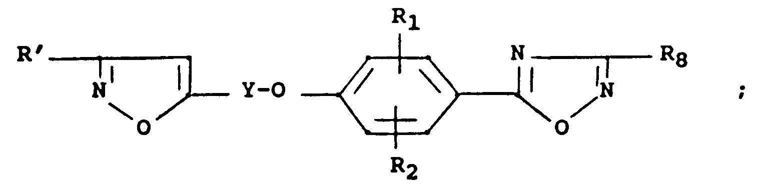
ii) reacting a compound of the formula 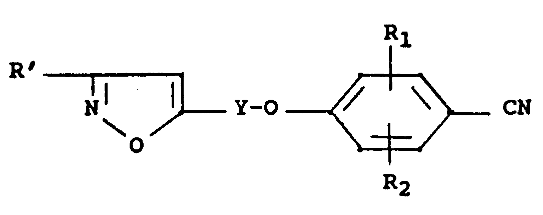 or the imino chloride derivative thereof with hydroxylamine, followed by reaction of the resulting product with an anhydride of the formula (R₈CO)₂O to produce a compound of the formula 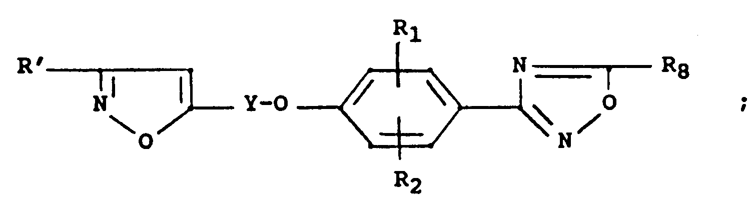
iii) reacting a compound of the formula 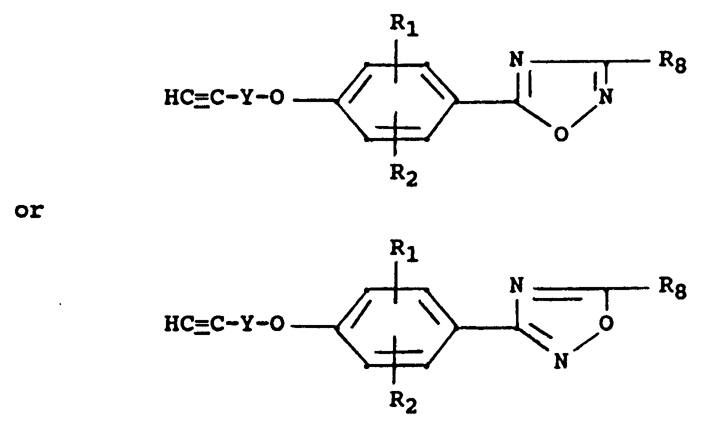 with a compound of the formula R'CH₂NO₂ and phenylisocyanate;
iv) reacting a compound of the formula 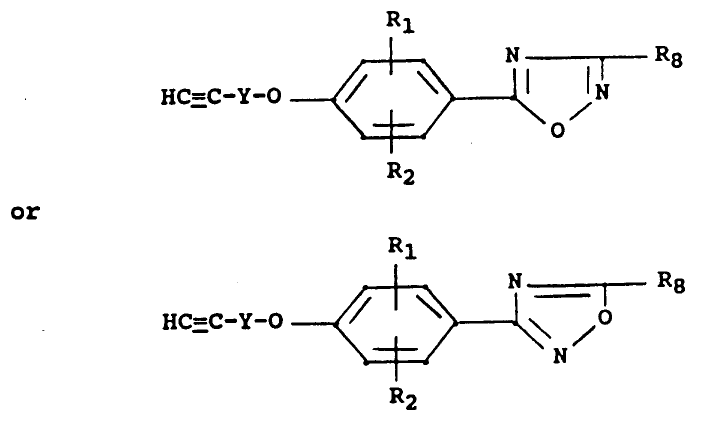 with a compound of the formula R'CH=NOH and N-halosuccinimide where halo is bromo or chloro;
(v) reacting a compound of the formula 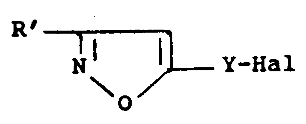 with a compound of the formula 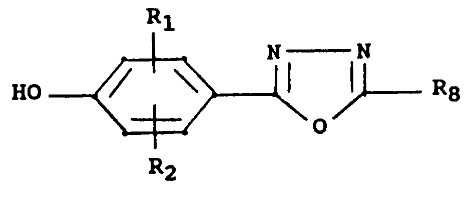 where Hal is chlorine, bromine or iodine;
(vi) reacting a compound of the formula 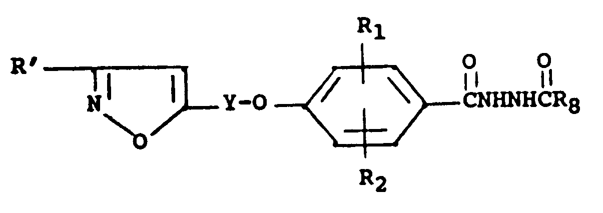 with hexamethyldisilazane in the presence of tetrabutylammonium trifluoride or
(vii) reacting a compound of the formula 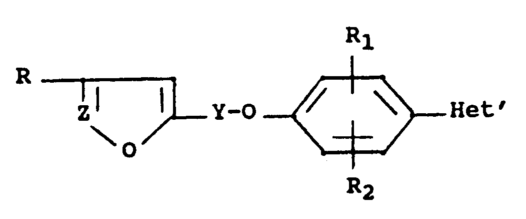 wherein:
Y is an alkylene bridge of 3-9 carbon atoms;
Z is N;
R is alkyl of 1-5 carbon atoms;
R₁ and R₂ are hydrogen, halogen, C₁₋₅ alkyl, C₁₋₅ alkoxy, nitro, lower-alkoxycarbonyl or trifluoromethyl; and
Het' is 1H-tetrazol-5-yl
with an alkanoic acid chloride of the formula R₈CO-Hal'' where Hal'' is chlorine or bromine, or an alkanoic acid anhydride of the formula (R₈CO)₂O.

2. A process according to claim 1, wherein the product is of the formula 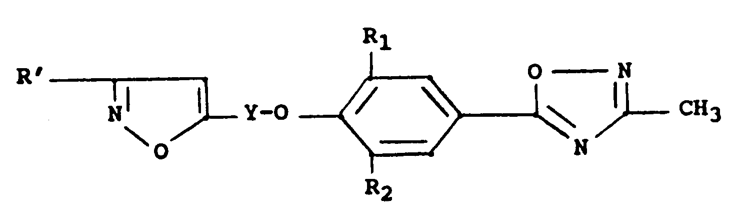 wherein Y is an alkylene bridge of 3-9 carbon atoms; R' is alkyl of 1-5 carbon atoms; and R₁ and R₂ are hydrogen, halogen or C₁₋₅ alkyl.

3. A process according to claim 2, wherein the product is selected from 5-{5-[2,6-dimethyl-4-(3-methyl-1,2,4-oxadiazol-5-yl)phenoxy]pentyl}-3-methylisoxazole, 5-{3-[2,6-dimethyl-4-(3-methyl-1,2,4-oxadiazol-5-yl)phenoxy]propyl}-3-methylisoxazole, 5-{3-[2,6-dimethyl-4-(3-methyl-1,2,4-oxadiazol-5-yl)phenoxy]propyl}-3-ethylisoxazole, and 5-{3-[2,6-dimethyl-4-(3-methyl-1,2,4-oxadiazol-5-yl)phenoxy]propyl}-3-propylisoxazole.

4. A process according to claim 1, wherein the product is of the formula 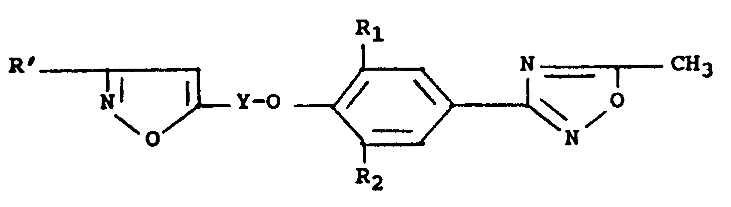 wherein Y is an alkylene bridge of 3-9 carbon atoms; R' is alkyl of 1-5 carbon atoms; and R₁ and R₂ are hydrogen, halogen or C₁₋₅ alkyl.

5. A process according to claim 4, wherein the product is selected from 5-{5-[2,6-dimethyl-4-(5-methyl-1,2,4-oxadiazol-3-yl)phenoxy]pentyl}-3-methylisoxazole, 5-{3-[2,6-dimethyl-4-(5-methyl-1,2,4-oxadiazol-3-yl)phenoxy]propyl}-3-methylisoxazole, and 5-{3-[2,6-dimethyl-4-(5-methyl-1,2,4-oxadiazol-3-yl)phenoxy] propyl}-3-ethylisoxazole.

6. A process according to claim 1, wherein the product is of the formula 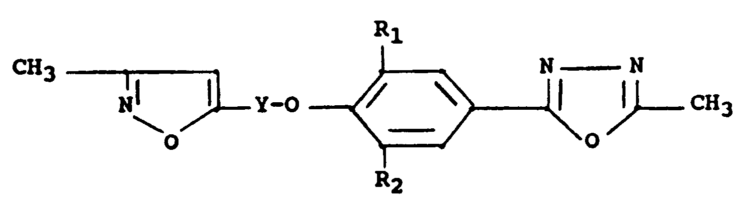 wherein Y, R₁ and R₂ have the meanings given in claim 1.

7. A process according to claim 6, wherein the product is selected from 5-{5-[2,6-dichloro-4-(5-methyl-1,3,4-oxadiazol-2-yl)phenoxy]pentyl}-3-methylisoxazole, and 5-{3-[2,6-dichloro-4-(5-methyl-1,3,4-oxadiazol-2-yl)phenoxy]propyl}-3-methylisoxazole.

8. Use of a compound according to any one of claims 1-7 as a non therapeutic antiviral agent.

9. Use of a compound according to any one of claims 1-7 for preparing a medicament to combat viral infections.

## Claims (Claims for the following Contracting State(s): GR)

1. A process for preparing compound of the formula 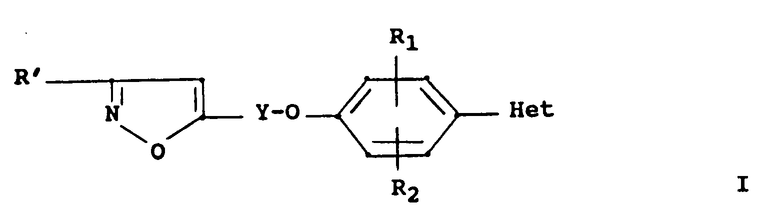 wherein:
Het is selected from 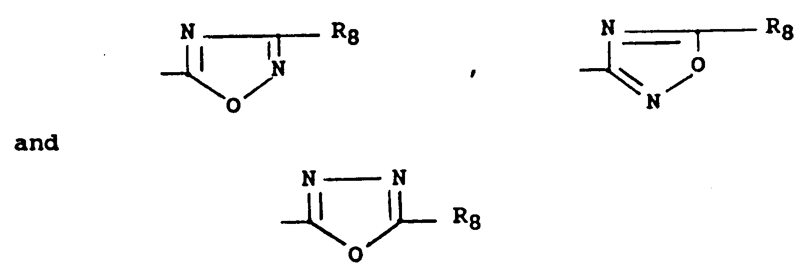 Y is an alkylene bridge of 3-9 carbon atoms;
R' is C₁₋₅ alkyl or hydroxy-C₁₋₅ alkyl atoms;
R₁ and R₂ are hydrogen, halogen, C₁₋₅ alkyl, C₁₋₅ alkoxy, nitro, C₁₋₅ alkoxycarbonyl or trifluoromethyl; and
R₈ is alkyl of 1-5 carbon atoms;
or pharmaceutically acceptable acid-addition salts thereof by one of the following reactions:
i) reacting a compound of the formula 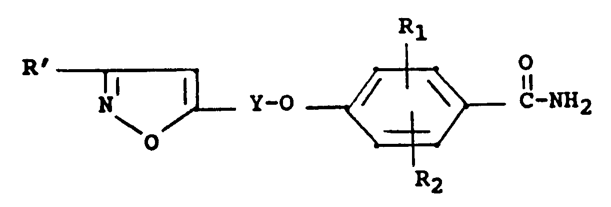 with a compound of the formula (CH₃)₂N-C(OCH₃)₂-R₈, followed by reaction of the resulting product with hydroxylamine in acetic acid solution to produce a compound of the formula 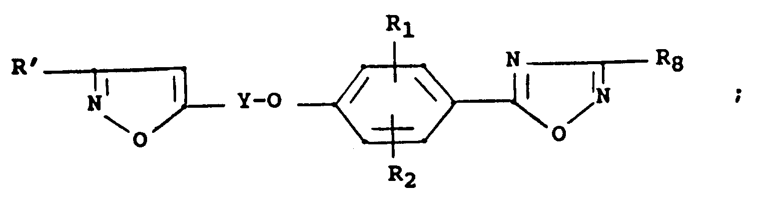
ii) reacting a compound of the formula 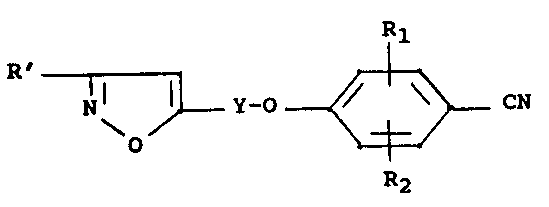 or the imino chloride derivative thereof with hydroxylamine, followed by reaction of the resulting product with an anhydride of the formula (R₈CO)₂O to produce a compound of the formula 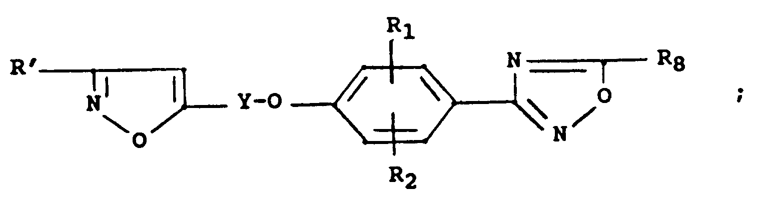
iii) reacting a compound of the formula 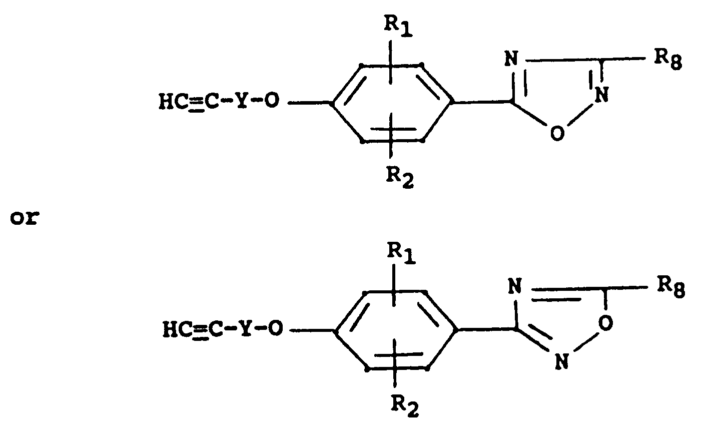 with a compound of the formula R'CH₂NO₂ and phenylisocyanate;
iv) reacting a compound of the formula 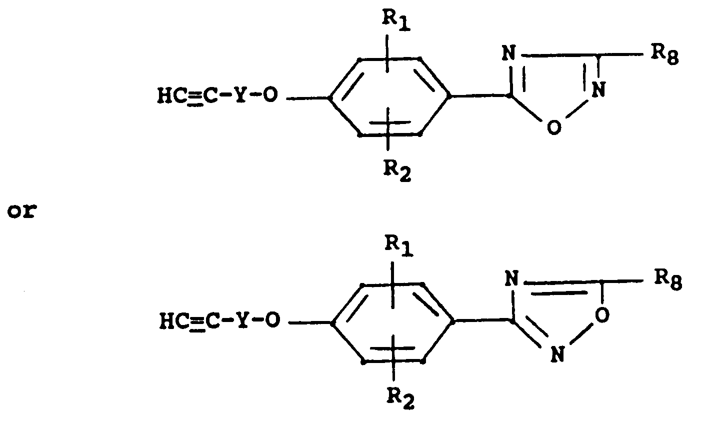 with a compound of the formula R'CH=NOH and N-halosuccinimide where halo is bromo or chloro;
(v) reacting a compound of the formula 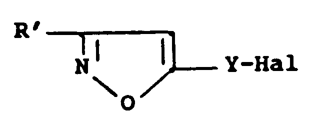 with a compound of the formula 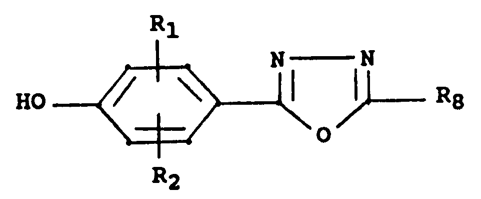 where Hal is chlorine, bromine or iodine;
(vi) reacting a compound of the formula 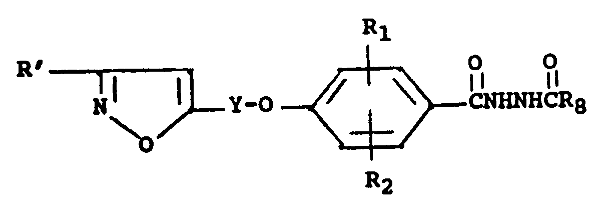 with hexamethyldisilazane in the presence of tetrabutylammonium trifluoride or
(vii) reacting a compound of the formula 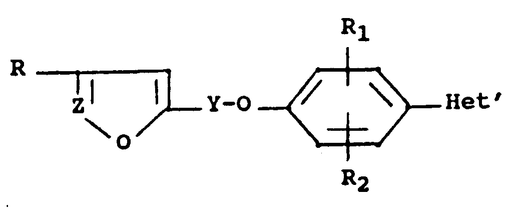 wherein:
Y is an alkylene bridge of 3-9 carbon atoms;
Z is N;
R is alkyl of 1-5 carbon atoms;
R₁ and R₂ are hydrogen, halogen, C₁₋₅ alkyl, C₁₋₅ alkoxy, nitro, lower-alkoxycarbonyl or trifluoromethyl; and
Het' is 1H-tetrazol-5-yl
with an alkanoic acid chloride of the formula R₈CO-Hal'' where Hal'' is chlorine or bromine, or an alkanoic acid anhydride of the formula (R₈CO)₂O.

2. A process according to claim 1, wherein the product is of the formula 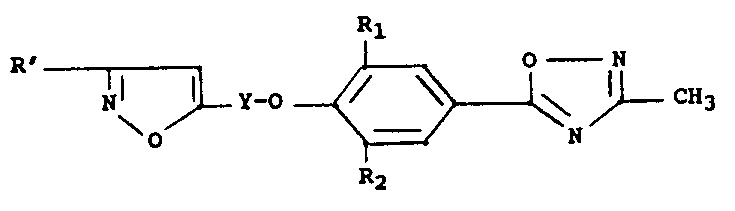 wherein Y is an alkylene bridge of 3-9 carbon atoms; R' is alkyl of 1-5 carbon atoms; and R₁ and R₂ are hydrogen, halogen or C₁₋₅ alkyl.

3. A process according to claim 2, wherein the product is selected from 5-{5-[2,6-dimethyl-4-(3-methyl-1,2,4-oxadiazol-5-yl)phenoxy]pentyl}-3-methylisoxazole, 5-{3-[2,6-dimethyl-4-(3-methyl-1,2,4-oxadiazol-5-yl)phenoxy]propyl}-3-methylisoxazole, 5-{3-[2,6-dimethyl-4-(3-methyl-1,2,4-oxadiazol-5-yl)phenoxy]propyl}-3-ethylisoxazole, and 5-{3-[2,6-dimethyl-4-(3-methyl-1,2,4-oxadiazol-5-yl)phenoxy]propyl}-3-propylisoxazole.

4. A process according to claim 1, wherein the product is of the formula 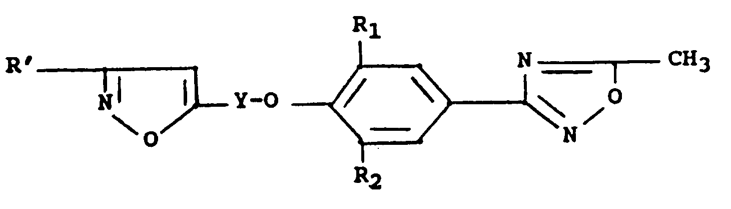 wherein Y is an alkylene bridge of 3-9 carbon atoms; R' is alkyl of 1-5 carbon atoms; and R₁ and R₂ are hydrogen, halogen or C₁₋₅ alkyl.

5. A process according to claim 4, wherein the product is selected from 5-{5-[2,6-dimethyl-4-(5-methyl-1,2,4-oxadiazol-3-yl)phenoxy]pentyl}-3-methylisoxazole, 5-{3-[2,6-dimethyl-4-(5-methyl-1,2,4-oxadiazol-3-yl)phenoxy]propyl}-3-methylisoxazole, and 5-{3-[2,6-dimethyl-4-(5-methyl-1,2,4-oxadiazol-3-yl)phenoxy] propyl}-3-ethylisoxazole.

6. A process according to claim 1, wherein the product is of the formula 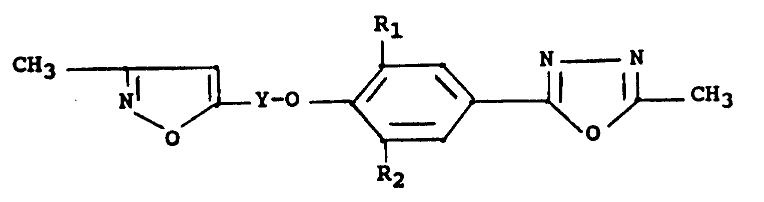 wherein Y, R₁ and R₂ have the meanings given in claim 1.

7. A process according to claim 6, wherein the product is selected from 5-{5-[2,6-dichloro-4-(5-methyl-1,3,4-oxadiazol-2-yl)phenoxy]pentyl}-3-methylisoxazole, and 5-{3-[2,6-dichloro-4-(5-methyl-1,3,4-oxadiazol-2-yl)phenoxy]propyl}-3-methylisoxazole.

8. Use of a compound according to any one of claims 1-7 as a non therapeutic antiviral agent.

9. Use of a compound according to any one of claims 1-7 for preparing a medicament to combat viral infections.

## Patentansprüche (Patentansprüche für folgende(n) Vertragsstaat(en): DK, DE, GB, FR, IT, NL, SE, LI, CH, BE, AT)

**1**. Verfahren zum Herstellen einer Verbindung der Formel 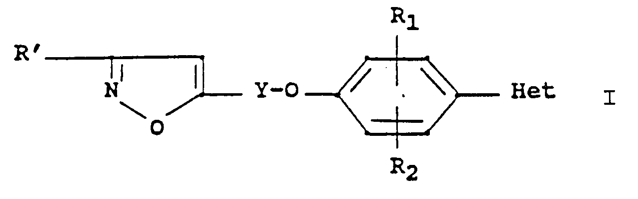 worin sind:
Het wird ausgewählt aus 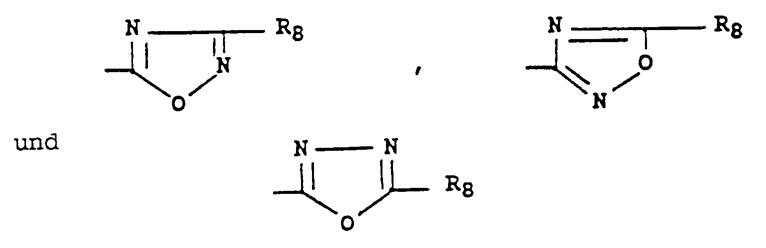 Y eine Alkylenbrücke mit 3 bis 9 Kohlenstoffatomen;
R' C₁ ... C₅-Alkyl- oder Hydroxy-C₁ ... C₅-alkylatome;
R₁ und R₂ Wasserstoff, Halogen, C₁ ... C₅-Alkyl, C₁ ... C₅-Alkoxy, Nitro, C₁ ... C₅-Alkoxycarbonyl oder Trifluormethyl; und
R₈ Alkyl mit 1 bis 5 Kohlenstoffatomen;
oder ein pharmazeutisch zulässiges Säure-Anlagerungssalz davon.

**2**. Verbindung nach Anspruch 1 der Formel: 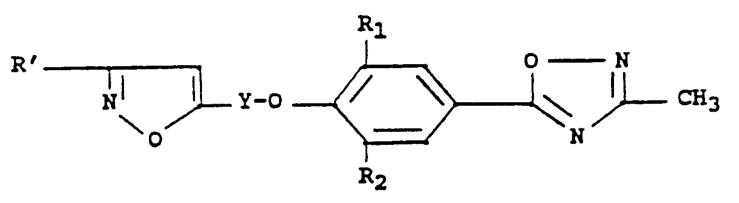 worin sind:
Y eine Alkylenbrücke mit 3 bis 9 Kohlenstoffatomen;
R' Alkyl mit 1 bis 5 Kohlenstoffatomen und
R₁ und R₂ Wasserstoff, Halogen oder C₁ ...C₅-Alkyl.

**3**. Verbindung nach Anspruch 2, ausgewählt aus: 5-{5-[2,6-Dimethyl-4-(3-methyl-1,2,4-oxadiazol-5-yl)phenoxy]pentyl}-3-methylisoxazol,
5-{3-[2,6-Dimethyl-4-(3-methyl-1,2,4-oxadiazol-5-yl)phenoxy]propyl}-3-methylisoxazol,
5-{3-[2,6-dimethyl-4-(3-methyl-1,2,4-oxadiazol-5-yl)phenoxy]propyl}-3-ethylisoxazol und
5-{3-[2,6-Dimethyl-4-(3-methyl-1,2,4-oxadiazol-5-yl)phenoxy]propyl}-3-propylisoxazol.

**4**. Verbindung nach Anspruch 1 der Formel: 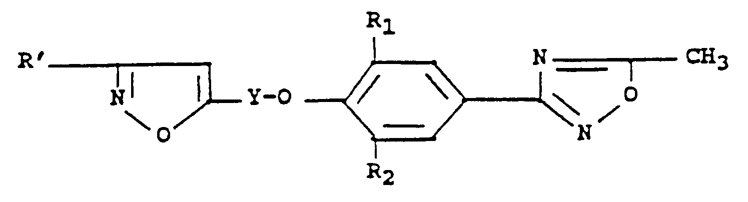 worin sind:
Y eine Alkylen-Brücke mit 3 bis 9 Kohlenstoffatomen;
R' Alkyl mit 1 bis 5 Kohlenstoffatomen und
R₁ und R₂ Wasserstoff, Halogen oder C₁ ... C₅-Alkyl.

**5**. Verbindung nach Anspruch 4, ausgewählt aus:
5-{5-[2,6-Dimethyl-4-(5-methyl-1,2,4-oxadiazol-3-yl)phenoxy]pentyl}-3-methylisoxazol,
5-{3-[2,6-Dimethyl-4-(5-methyl-1,2,4-oxadiazol-3-yl)phenoxy]propyl}-3-methylisoxazol und
5-{3-[2,6-Dimethyl-4-(5-methyl-1,2,4-oxadiazol-3-yl)phenoxy]propyl}-3-ethylisoxazol.

**6.** Verbindung nach Anspruch 1 der Formel: 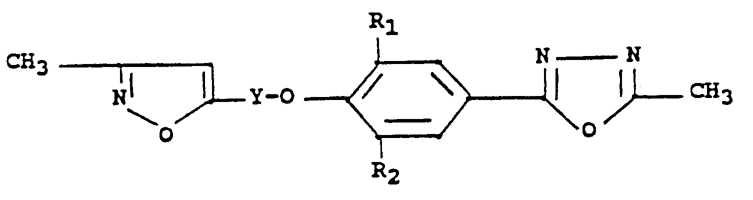 worin Y, R₁ und R₂ die in Anspruch 1 festgelegten Bedeutungen haben.

**7**. Verbindung nach Anspruch 6, ausgewählt aus:
5-{5-[2,6-Dichlor-4-(5-methyl-1,3,4-oxadiazol-2-yl)phenoxy]pentyl}-3-methylisoxazol und
5-{3-[2,6-Dichlor-4-(5-methyl-1,3,4-oxadiazol-2-yl)phenoxy]propyl}-3-methylisoxazol.

**8**. Zusammensetzung zur Bekämpfung von Pocornaviren, welche eine antiviral wirksame Menge einer Verbindung nach einem der vorgenannten Ansprüche umfaßt, und zwar in Zumischung mit einem geeigneten Träger oder Verdünnungsmittel.

**9**. Zusammensetzung nach Anspruch 8 zur Bekämpfung von Rhinoviren.

**10**. Verbindung der Formel 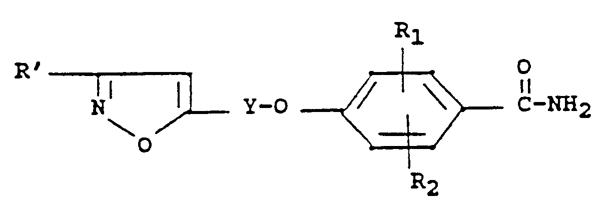 worin Y eine Alkylenbrücke mit 3 bis 9 Kohlenstoffatomen ist, R' ist C₁ ... C₅-Alkyl oder -Hydroxyalkyl mit 1 bis 5 Kohlenstoffatomen und R₁ und R₂ sind Halogen oder C₁ ... C₅-Alkyl.

**11**. Verbindung nach Anspruch 10, ausgewählt aus:
3,5-Dimethyl-4-[3-(3-methylisoxazol-5-yl)propyloxy]benzamid und
3,5-Dimethyl-4-[3-(3-ethylisoxazol-5-yl)propyloxy]benzamid.

**12**. Verfahren zum Herstellen einer Verbindung nach Anspruch 1, umfassend das Umsetzen einer Verbindung der Formel 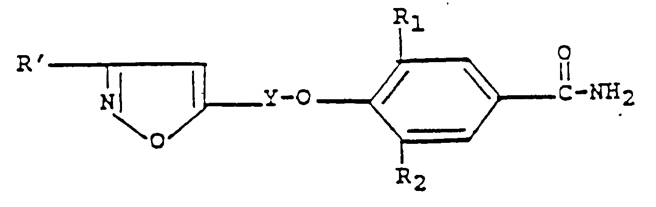 mit einer Verbindung der Formel (CH₃)₂N-C(OCH₃)₂-R₈, gefolgt von einem Umsetzen des resultierenden Produkts mit Hydroxlamin in Essigsäurelösung, um eine Verbindung der Formel 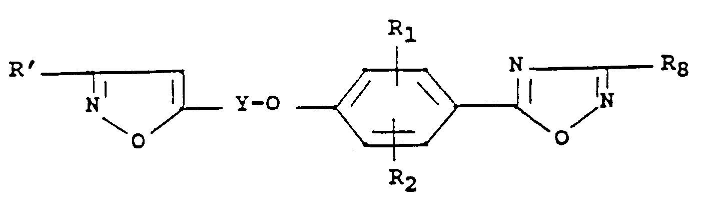 zu erzeugen;

**13**. Verfahren zum Herstellen einer Verbindung nach Anspruch 1, umfassend das Umsetzen einer Verbindung der Formel 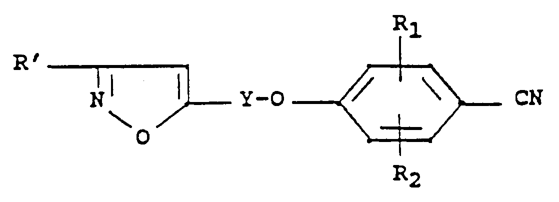 oder des Iminochlorid-Derivats davon mit Hydroxylamin, gefolgt von einem Umsetzen des resultierenden Produkts mit einem Anhydrid der Formel (R₈CO)₂O, um eine Verbindung der Formel 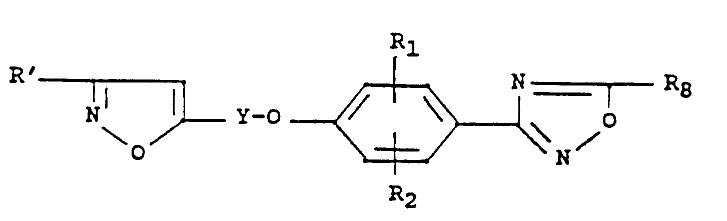 zu erzeugen.

**14**. Verfahren zum Herstellen einer Verbindung nach Anspruch 1, umfassend das Umsetzen einer Verbindung der Formel 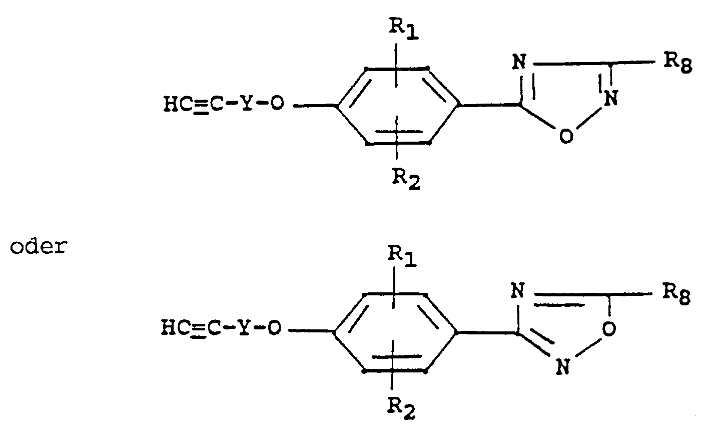 mit einer Verbindung der Formel R'CH₂NO₂ und Phenylisocyanat.

**15**. Verfahren zum Herstellen einer Verbindung nach Anspruch 1, umfassend das Umsetzen einer Verbindung der Formel 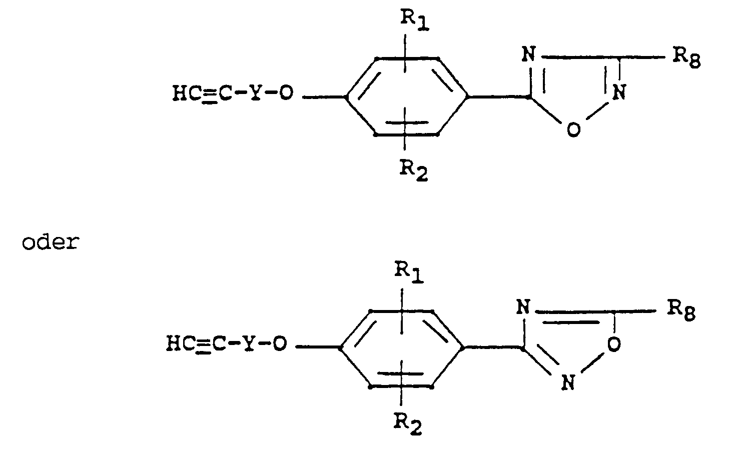 mit einer Verbindung der Formel R'CH=NOH und N-Halogensuccinimid, worin Halogen Brom oder Chlor ist.

**16**. Verfahren zum Herstellen einer Verbindung nach Anspruch 1, umfassend das Umsetzen einer Verbindung der Formel 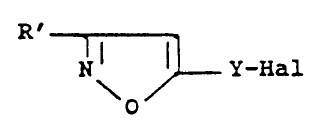 mit einer Verbindung der Formel 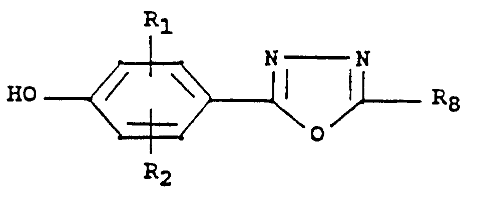 worin Hal Chlor, Brom oder Iod ist.

**17**. Verfahren zum Herstellen einer Verbindung nach Anspruch 1, umfassend das Umsetzen einer Verbindung der Formel 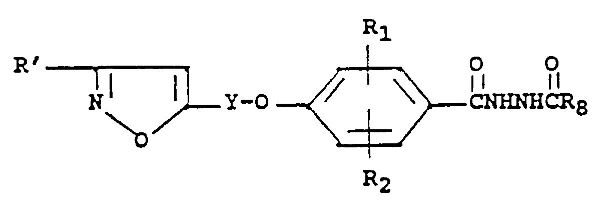 mit Hexamethyldisilazan in Anwesenheit von Tetrabutylammoniumtrifluorid.

**18**. Verfahren zum Herstellen einer Verbindung nach Anspruch 1, worin R' Alkyl mit 1 bis 5 Kohlenstoffatomen und Het 5-C₁ ... C₅-Alkyl-1,3,4-oxadia-zolyl sind, umfassend das Umsetzen einer Verbindung der Formel worin sind:
Y eine Alkylen-Brücke mit 3 bis 9 Kohlenstoffatomen;
Z N;
R Alkyl mit 1 bis 5 Kohlenstoffatomen;
R₁ und R₂ sind Wasserstoff, Halogen, C₁ ... C₅-Alkyl, C₁ ... C₅-Alkoxy, Nitro, C₂ ... C₄-Alkoxycarbonyl oder Trifluormethyl; und
Het' 1H-Tetrazol-5-yl
mit einem Alkansäurechlorid der Formel R₈CO-Hal'', worin Hal'' Chlor oder Brom oder ein Alkansäureanhydrid der Formel (R₈CO)₂O ist.

**20**. Verwendung einer Verbindung nach einem der Ansprüche 1 bis 7 als ein nicht-therapeutisches antivirales Mittel.

**21**. Verwendung einer Verbindung nach einem der Ansprüche 1 bis 7 zur Zubereitung eines Medikamentes zur Bekämpfung viraler Infektionen.

## Patentansprüche (Patentansprüche für folgende(n) Vertragsstaat(en): ES)

1. Verfahren zum Herstellen einer Verbindung der Formel 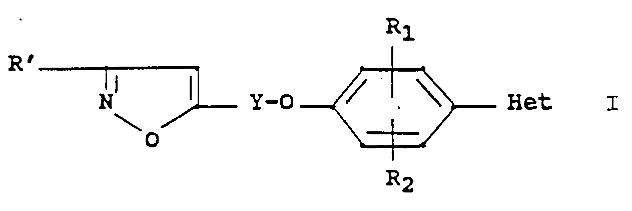 worin sind:
Het wird ausgewählt aus 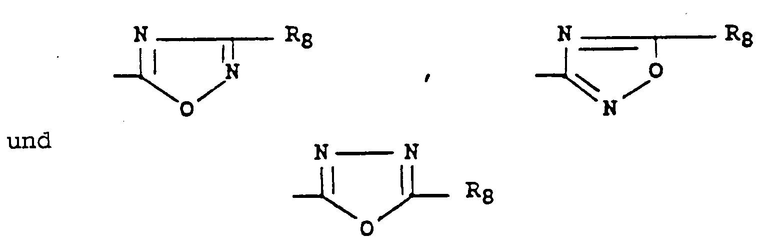 Y eine Alkylenbrücke mit 3 bis 9 Kohlenstoffatomen;
R' C₁ ... C₅-Alkyl- oder Hydroxy-C₁ ... C₅-alkylatome;
R₁ und R₂ Wasserstoff, Halogen, C₁ ... C₅-Alkyl, C₁ ... C₅-Alkoxy, Nitro, C₁...C₅-Alkoxycarbonyl oder Trifluormethyl; und
R₈ Alkyl mit 1 bis 5 Kohlenstoffatomen;
oder ein pharmazeutisch zulässiges Säure-Anlagerungssalz davon
mit Hilfe einer der folgenden Reaktionen:
i) Umsetzen einer Verbindung der Formel 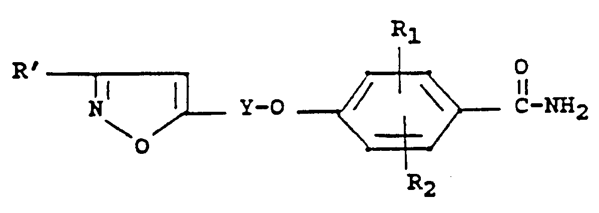 mit einer Verbindung der Formel (CH₃)₂N-C(OCH₃)₂-R₈, gefolgt von einem Umsetzen des resultierenden Produkts mit Hydroxylamin in Essigsäurelösung, um eine Verbindung der Formel 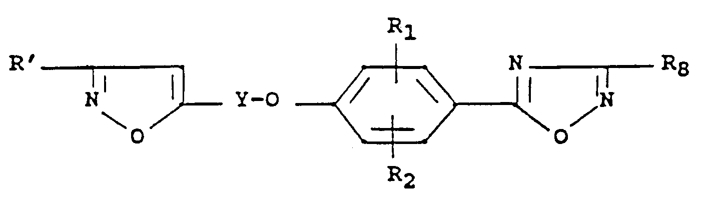 zu erzeugen;
ii) Umsetzen einer Verbindung der Formel 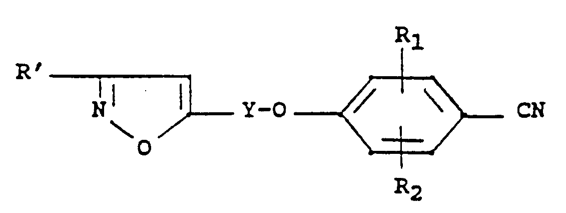 oder des Iminochlorid-Derivats davon mit Hydroxylamin, gefolgt von einem Umsetzen des resultierenden Produkts mit einem Anhydrid der Formel (R₈CO)₂O, um eine Verbindung der Formel 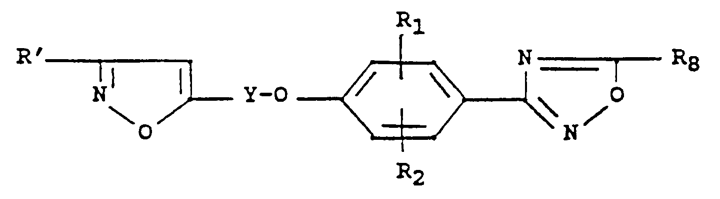 zu erzeugen;
iii) Umsetzen einer Verbindung der Formel 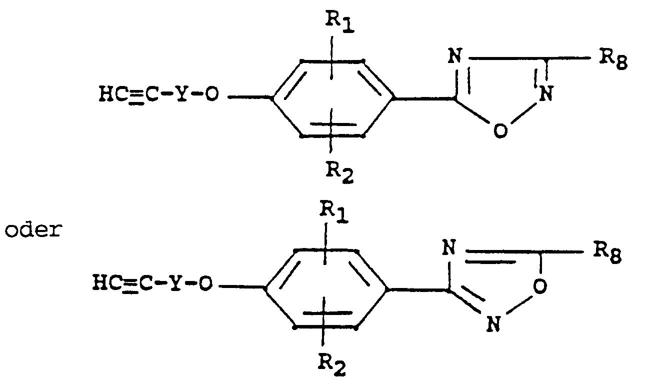 mit einer Verbindung der Formel R'CH₂NO₂ und Phenylisocyanat;
iv) Umsetzen einer Verbindung der Formel 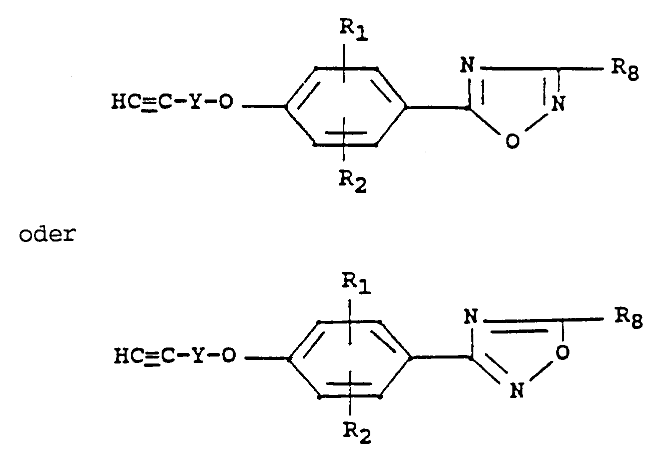 mit einer Verbindung der Formel R'CH=NOH und N-Halogensuccinimid, worin Halogen Brom oder Chlor ist;
(v) Umsetzen einer Verbindung der Formel 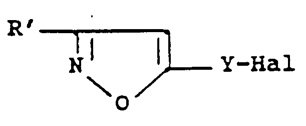 mit einer Verbindung der Formel 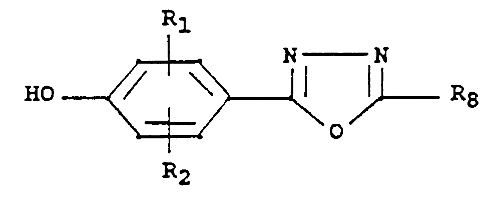 worin Hal Chlor, Brom oder Iod ist;
(vi) Umsetzen einer Verbindung der Formel 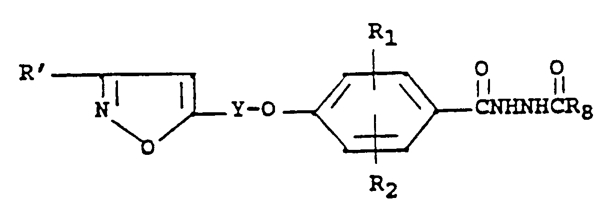 mit Hexamethyldisilazan in Anwesenheit von Tetrabutylammoniumtrifluorid oder
(vii) Umsetzen einer Verbindung der Formel 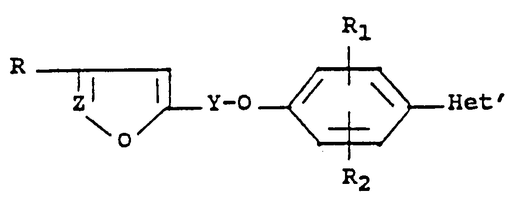 worin sind:
Y eine Alkylenbrücke mit 3 bis 9 Kohlenstoffatomen;
Z N;
R Alkyl mit 1 bis 5 Kohlenstoffatomen;
R₁ und R₂ sind Wasserstoff, Halogen, C₁ ... C₅-Alkyl, C₁...C₅-Alkoxy, Nitro, niederes Alkoxycarbonyl oder Trifluormethyl; und
Het' 1H-Tetrazol-5-yl
mit einem Alkansäurechlorid der Formel R₈CO-Hal'', worin Hal'' Chlor oder Brom oder ein Alkansäureanhydrid der Formel (R₈CO)₂O ist.

2. Verfahren nach Anspruch 1, bei welchem das Produkt die Formel hat: 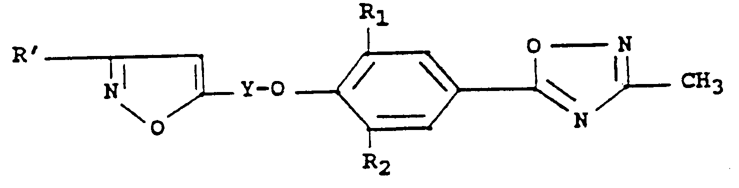 worin sind:
Y eine Alkylenbrücke mit 3 bis 9 Kohlenstoffatomen;
R' Alkyl mit 1 bis 5 Kohlenstoffatomen und
R₁ und R₂ Wasserstoff, Halogen oder C₁ ...C₅-Alkyl.

3. Verfahren nach Anspruch 2, bei welchem das Produkt ausgewählt wird aus:
5-{5-[2,6-Dimethyl-4-(3-methyl-1,2,4-oxadiazol-5-yl)phenoxy]pentyl}-3-methylisoxazol,
5-{3-[2,6-Dimethyl-4-(3-methyl-1,2,4-oxadiazol-5-yl)phenoxy]propyl}-3-methylisoxazol,
5-{3-[2,6-dimethyl-4-(3-methyl-1,2,4-oxadiazol-5-yl)phenoxy]propyl}-3-ethylisoxazol und
5-{3-[2,6-Dimethyl-4-(3-methyl-1,2,4-oxadiazol-5-yl)phenoxy]propyl}-3-propylisoxazol.

4. Verfahren nach Anspruch 1, bei welchem das Produkt die Formel hat: 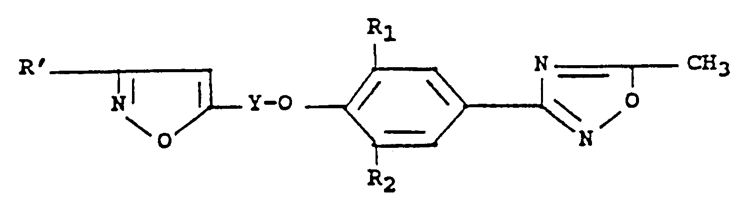 worin sind:
Y eine Alkylenbrücke mit 3 bis 9 Kohlenstoffatomen;
R' Alkyl mit 1 bis 5 Kohlenstoffatomen und
R₁ und R₂ Wasserstoff, Halogen oder C₁ ... C₅-Alkyl.

5. Verfahren nach Anspruch 4, bei welchem das Produkt ausgewählt wird aus:
5-{5-[2,6-Dimethyl-4-(5-methyl-1,2,4-oxadiazol-3-yl)phenoxy]pentyl}-3-methylisoxazol,
5-{3-[2,6-Dimethyl-4-(5-methyl-1,2,4-oxadiazol-3-yl)phenoxy]propyl}-3-methylisoxazol und
5-{3-[2,6-Dimethyl-4-(5-methyl-1,2,4-oxadiazol-3-yl)phenoxy]propyl}-3-ethylisoxazol.

6. Verfahren nach Anspruch 1, bei welchem das Produkt die Formel hat: 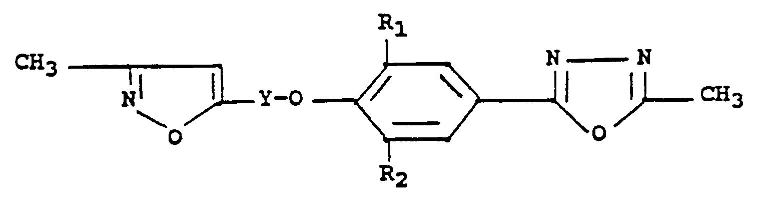 worin Y, R₁ und R₂ die in Anspruch 1 festgelegten Bedeutungen haben.

7. Verfahren nach Anspruch 6, bei welchem das Produkt ausgewählt wird aus:
5-{5-[2,6-Dichlor-4-(5-methyl-1,3,4-oxadiazol-2-yl)phenoxy]pentyl}-3-methylisoxazol und
5-{3-[2,6-Dichlor-4-(5-methyl-1,3,4-oxadiazol-2-yl)phenoxy]propyl}-3-methylisoxazol.

8. Verwendung einer Verbindung nach einem der Ansprüche 1 bis 7 als ein nicht-therapeutisches antivirales Mittel.

9. Verwendung einer Verbindung nach einem der Ansprüche 1 bis 7 zur Zubereitung eines Medikamentes zur Bekämpfung viraler Infektionen.

## Patentansprüche (Patentansprüche für folgende(n) Vertragsstaat(en): GR)

1. Verfahren zum Herstellen einer Verbindung der Formel 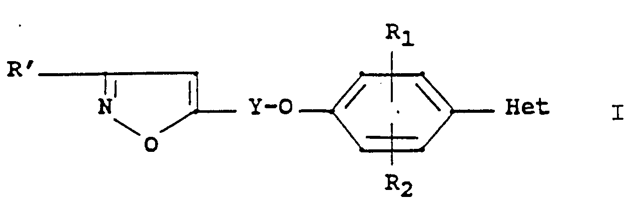 worin sind:
Het wird ausgewählt aus 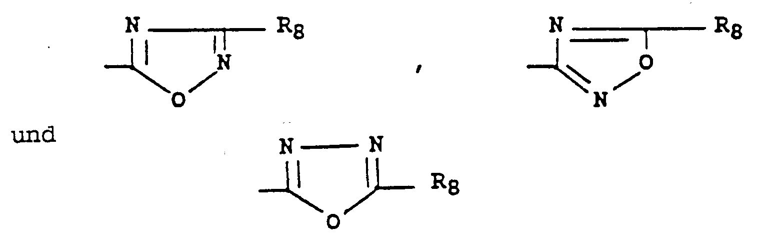 Y eine Alkylenbrücke mit 3 bis 9 Kohlenstoffatomen;
R' C₁ ... C₅-Alkyl- oder Hydroxy-C₁ ... C₅-alkylatome;
R₁ und R₂ Wasserstoff, Halogen, C₁ ... C₅-Alkyl, C₁ ... C₅-Alkoxy, Nitro, C₁...C₅-Alkoxycarbonyl oder Trifluormethyl; und
R₈ Alkyl mit 1 bis 5 Kohlenstoffatomen;
oder ein pharmazeutisch zulässiges Säure-Anlagerungssalz davon
mit Hilfe einer der folgenden Reaktionen:
i) Umsetzen einer Verbindung der Formel 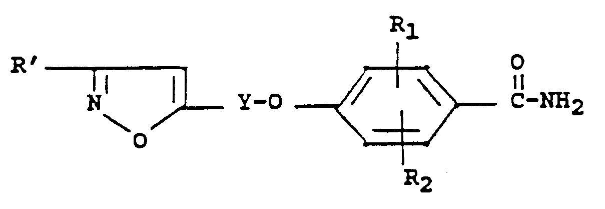 mit einer Verbindung der Formel (CH₃)₂N-C(OCH₃)₂-R₈, gefolgt von einem Umsetzen des resultierenden Produkts mit Hydroxylamin in Essigsäurelösung, um eine Verbindung der Formel 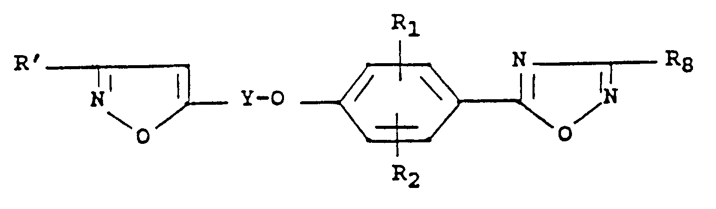 zu erzeugen;
ii) Umsetzen einer Verbindung der Formel 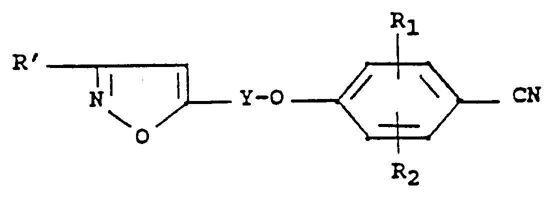 oder des Iminochlorid-Derivats davon mit Hydroxylamin, gefolgt von einem Umsetzen des resultierenden Produkts mit einem Anhydrid der Formel (R₈CO)₂O, um eine Verbindung der Formel 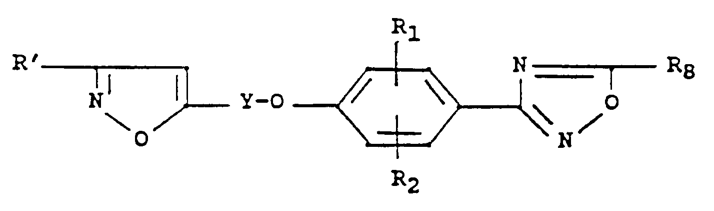 zu erzeugen;
iii) Umsetzen einer Verbindung der Formel 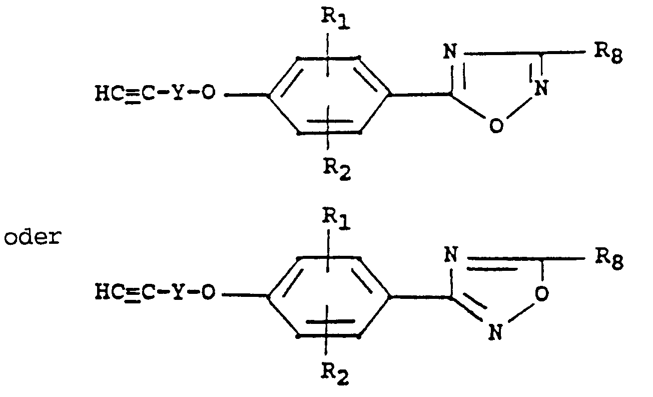 mit einer Verbindung der Formel R'CH₂NO₂ und Phenylisocyanat;
iv) Umsetzen einer Verbindung der Formel 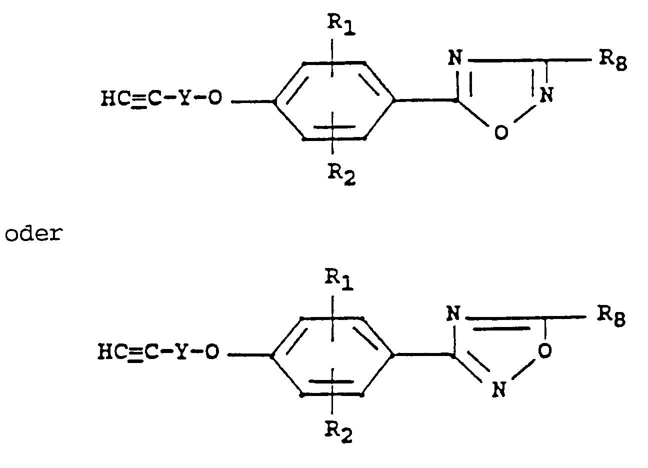 mit einer Verbindung der Formel R'CH=NOH und N-Halogensuccinimid, worin Halogen Brom oder Chlor ist;
(v) Umsetzen einer Verbindung der Formel 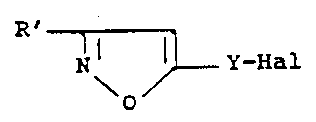 mit einer Verbindung der Formel 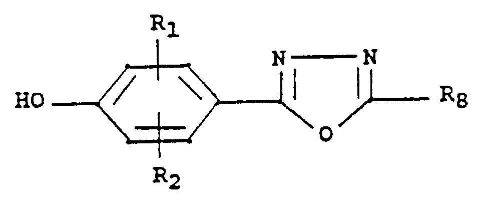 worin Hal Chlor, Brom oder Iod ist;
(vi) Umsetzen einer Verbindung der Formel 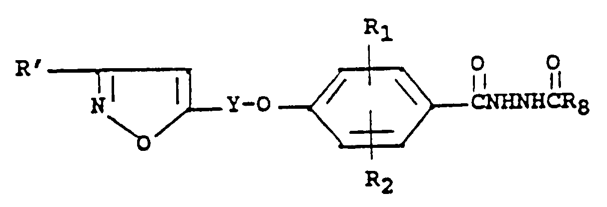 mit Hexamethyldisilazan in Anwesenheit von Tetrabutylammoniumtrifluorid oder
(vii) Umsetzen einer Verbindung der Formel 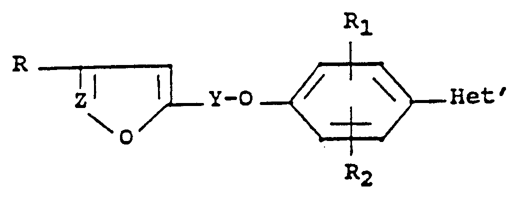 worin sind:
Y eine Alkylenbrücke mit 3 bis 9 Kohlenstoffatomen;
Z N;
R Alkyl mit 1 bis 5 Kohlenstoffatomen;
R₁ und R₂ sind Wasserstoff, Halogen, C₁ ... C₅-Alkyl, C₁ ... C₅-Alkoxy, Nitro, niederes Alkoxycarbonyl oder Trifluormethyl; und
Het' 1H-Tetrazol-5-yl
mit einem Alkansäurechlorid der Formel R₈CO-Hal'', worin Hal'' Chlor oder Brom oder ein Alkansäureanhydrid der Formel (R₈CO)₂O ist.

2. Verfahren nach Anspruch 1, bei welchem das Produkt die Formel hat: 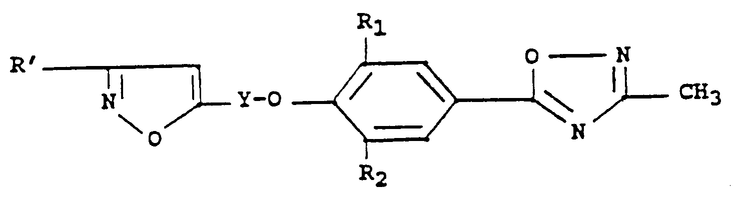 worin sind:
Y eine Alkylenbrücke mit 3 bis 9 Kohlenstoffatomen;
R' Alkyl mit 1 bis 5 Kohlenstoffatomen und
R₁ und R₂ Wasserstoff, Halogen oder C₁ ...C₅-Alkyl.

3. Verfahren nach Anspruch 2, bei welchem das Produkt ausgewählt wird aus:
5-{5-[2,6-Dimethyl-4-(3-methyl-1,2,4-oxadiazol-5-yl)phenoxy]pentyl}-3-methylisoxazol,
5-{3-[2,6-Dimethyl-4-(3-methyl-1,2,4-oxadiazol-5-yl)phenoxy]propyl}-3-methylisoxazol,
5-{3-[2,6-dimethyl-4-(3-methyl-1,2,4-oxadiazol-5-yl)phenoxy]propyl}-3-ethylisoxazol und
5-{3-[2,6-Dimethyl-4-(3-methyl-1,2,4-oxadiazol-5-yl)phenoxy]propyl}-3-propylisoxazol.

4. Verfahren nach Anspruch 1, bei welchem das Produkt die Formel hat: 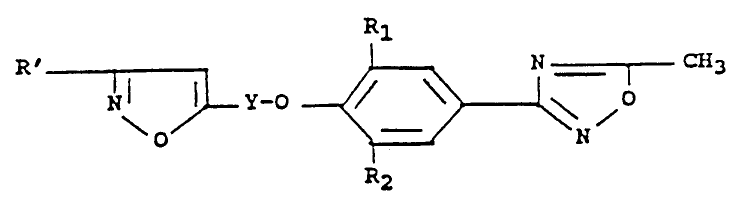 worin sind:
Y eine Alkylenbrücke mit 3 bis 9 Kohlenstoffatomen;
R' Alkyl mit 1 bis 5 Kohlenstoffatomen und
R₁ und R₂ Wasserstoff, Halogen oder C₁ ... C₅-Alkyl.

5. Verfahren nach Anspruch 4, bei welchem das Produkt ausgewählt wird aus:
5-{5-[2,6-Dimethyl-4-(5-methyl-1,2,4-oxadiazol-3-yl)phenoxy]pentyl}-3-methylisoxazol,
5-{3-[2,6-Dimethyl-4-(5-methyl-1,2,4-oxadiazol-3-yl)phenoxy]propyl}-3-methylisoxazol und
5-{3-[2,6-Dimethyl-4-(5-methyl-1,2,4-oxadiazol-3-yl)phenoxy]propyl}-3-ethylisoxazol.

6. Verfahren nach Anspruch 1, bei welchem das Produkt die Formel hat: 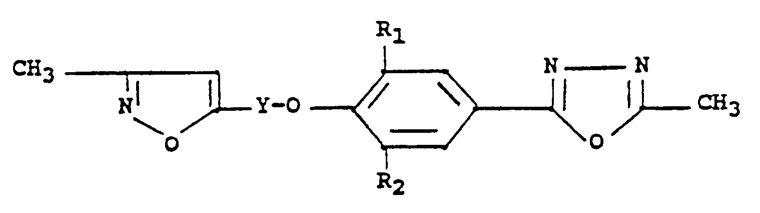 worin Y, R₁ und R₂ die in Anspruch 1 festgelegten Bedeutungen haben.

7. Verfahren nach Anspruch 6, bei welchem das Produkt ausgewählt wird aus:
5-{5-[2,6-Dichlor-4-(5-methyl-1,3,4-oxadiazol-2-yl)phenoxy]pentyl}-3-methylisoxazol und
5-{3-[2,6-Dichlor-4-(5-methyl-1,3,4-oxadiazol-2-yl)phenoxy]propyl}-3-methylisoxazol.

8. Verwendung einer Verbindung nach einem der Ansprüche 1 bis 7 als ein nicht-therapeutisches antivirales Mittel.

9. Verwendung einer Verbindung nach einem der Ansprüche 1 bis 7 zur Zubereitung eines Medikamentes zur Bekämpfung viraler Infektionen.

## Revendications (Revendications pour l'(les) Etat(s) contractant(s) suivant(s): DK, DE, GB, FR, IT, NL, SE, LI, CH, BE, AT)

1. Composé de formule 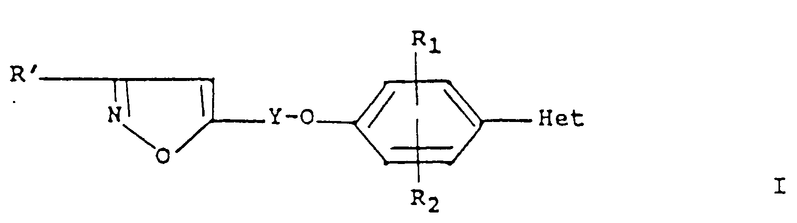 dans laquelle:
Het est choisi parmi 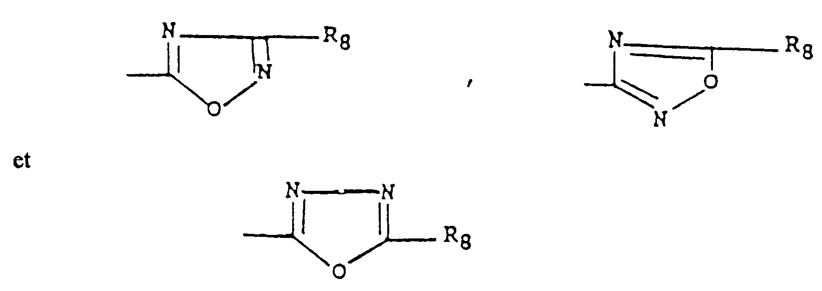 Y est un pont alkylène en C₃-C₉,
R' est un groupement alkyle en C₁-C₅ ou un groupement hydroxyalkyle en C₁-C₅,
R₁ et R₂ sont un atome d'hydrogène, un atome d'halogène, un groupement alkyle en C₁-C₅, un groupement alcoxy en C₁-C₅, un groupement nitro, un groupement (alcoxy en C₂-C₄)carbonyle ou un groupement trifluorométhyle et
R₈ est un groupement alkyle en C₁-C₅,
ou ses sels d'addition d'acide pharmaceutiquement acceptables.

2. Composé selon la revendication 1 de formule 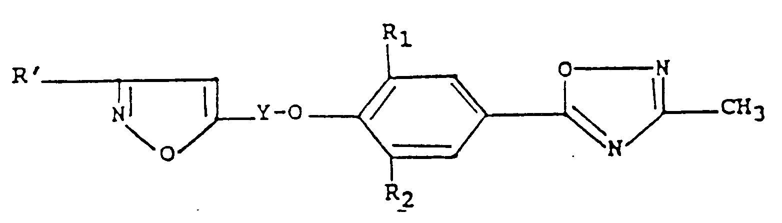 dans laquelle Y est un pont alkylène en C₃-C₉, R' est un groupement alkyle en C₁-C₅ et R₁ et R₂ sont un atome d'hydrogène, un atome d'halogène ou un groupement alkyle en C₁-C₅.

3. Composé selon la revendication 2, choisi parmi le 5-{5-[2,6-diméthyl-4-(3-méthyl-1,2,4-oxadiazol-5-yl)phénoxy]pentyl}-3-méthylisoxazole, le 5-{3-[2,6-diméthyl-4-(3-méthyl-1,2,4-oxadiazol-5-yl)phénoxy]propyl}-3-méthylisoxazole, le 5-{3-[2,6-diméthyl-4-(3-méthyl-1,2,4-oxadiazol-5-yl)phénoxy]propyl}-3-éthylisoxazole et le 5-{3-[2,6-diméthyl-4-(3-méthyl-1,2,4-oxadiazol-5-yl)phénoxy]propyl}-3-propylisoxazole.

4. Composé selon la revendication 1 de formule 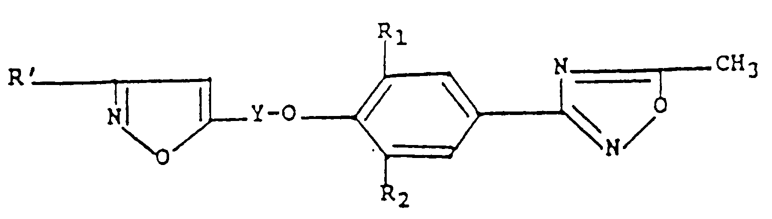 dans laquelle Y est un pont alkylène en C₃-C₉, R' est un groupement alkyle en C₁-C₅ et R₁ et R₂ sont un atome d'hydrogène, un atome d'halogène ou un groupement alkyle en C₁-C₅.

5. Composé selon la revendication 4, choisi parmi le 5-{5-[2,6-diméthyl-4-(5-méthyl-1,2,4-oxadiazol-3-yl)phénoxy]pentyl}-3-méthylisoxazole, le 5-{3-[2,6-diméthyl-4-(5-méthyl-1,2,4-oxadiazol-3-yl)phénoxy]propyl}-3-méthylisoxazole et le 5-{3-[2,6-diméthyl-4-(5-méthyl-1,2,4-oxadiazol-3-yl)phénoxy]propyl}-3-éthylisoxazole.

6. Composé selon la revendication 1 de formule 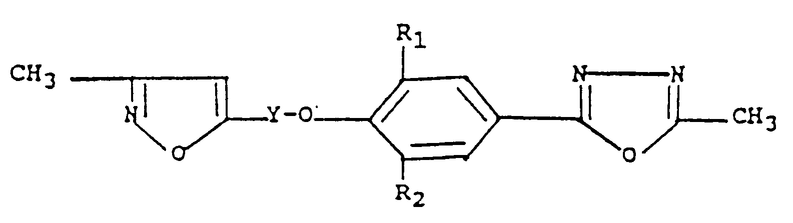 dans laquelle Y, R₁ et R₂ ont les significations données dans la revendication 1.

7. Composé selon la revendication 6, choisi parmi le 5-{5-[2,6-dichloro-4-(5-méthyl-1,3,4-oxadiazol-2-yl)phénoxy]pentyl}-3-méthylisoxazole et le 5-{3-[2,6-dichloro-4-(5-méthyl-1,3,4-oxadiazol-2-yl)phénoxy]propyl}-3-méthylisoxazole.

8. Composition destinée à lutter contre des picornavirus qui comprend une quantité active contre les virus d'un composé selon l'une quelconque des revendications précédentes, mélangé avec un véhicule ou un diluant adapté.

9. Composition selon la revendication 8, destinée à lutter contre des rhinovirus.

10. Composé de formule 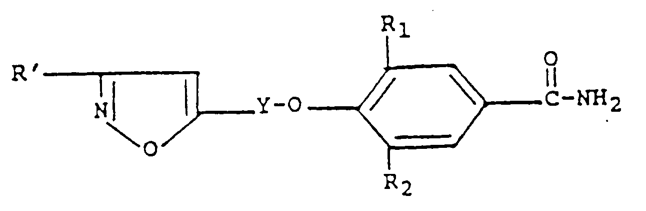 dans laquelle Y est un pont alkylène en C₃-C₉, R' est un groupement alkyle en C₁-C₅ ou un groupement hydroxyalkyle en C₁-C₅ et R₁ et R₂ sont un atome d'halogène ou un groupement alkyle en C₁-C₅.

11. Composé selon la revendication 10, choisi parmi le 3,5-diméthyl-4-[3-(3-méthylisoxazol-5-yl)propyloxy]benzamide et le 3,5-diméthyl-4-[3-(3-éthylisoxazol-5-yl)propyloxy]benzamide.

12. Procédé de préparation d'un composé selon la revendication 1, qui comprend la réaction d'un composé de formule 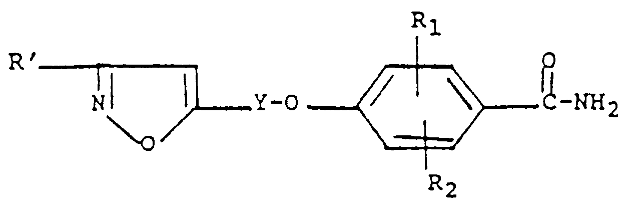 avec un composé de formule (CH₃)₂N-C(OCH₃)₂-R₈, suivie par la réaction du produit résultant avec de l'hydroxylamine en solution d'acide acétique pour produire un composé de formule

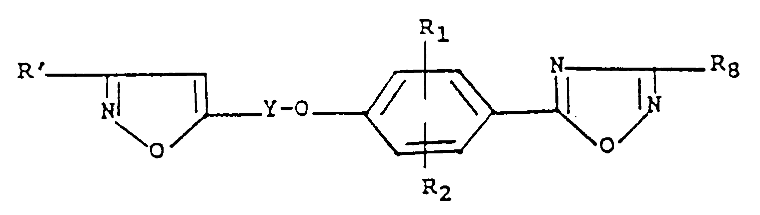

13. Procédé de préparation d'un composé selon la revendication 1, qui comprend la réaction d'un composé de formule 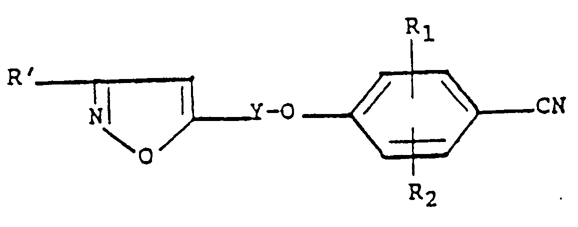 ou de son dérivé de type iminochlorure avec de l'hydroxylamine, suivie par la réaction du produit résultant avec un anhydride de formule (R₈CO)₂O pour produire un composé de formule

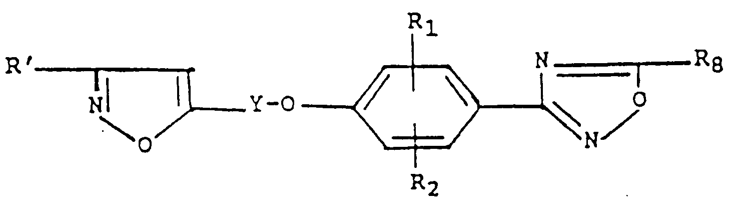

14. Procédé de préparation d'un composé selon la revendication 1, qui comprend la réaction d'un composé de formule 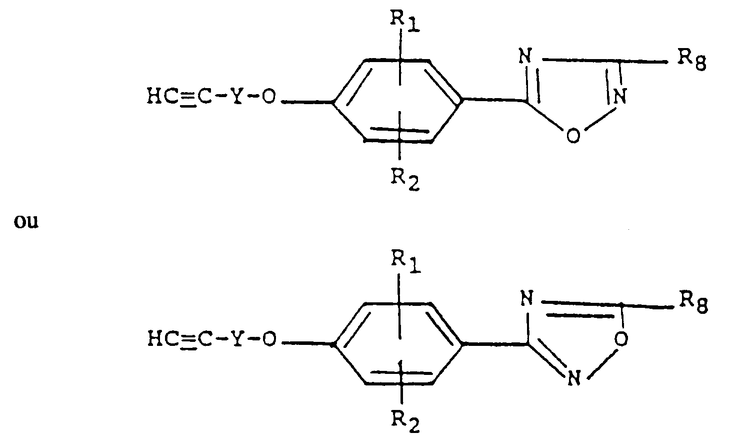 avec un composé de formule R'CH₂NO₂ et du phénylisocyanate.

15. Procédé de préparation d'un composé selon la revendication 1, qui comprend la réaction d'un composé de formule 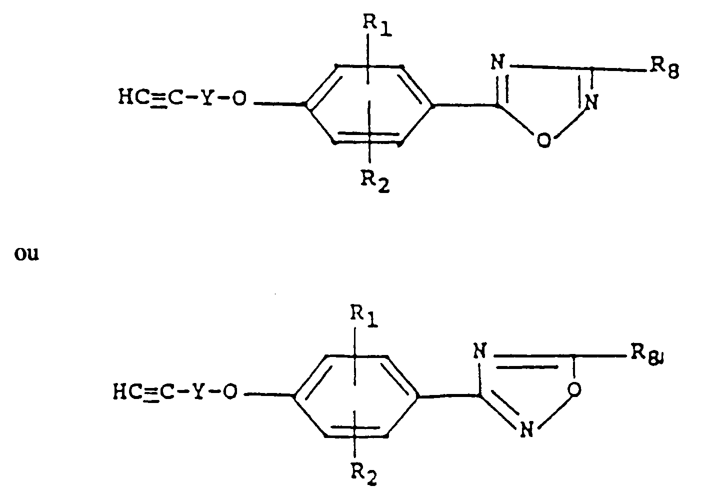 avec un composé de formule R'CH=NOH et du N-halogénosuccinimide pour lequel l'atome d'halogène est un atome de brome ou un atome de chlore.

16. Procédé de préparation d'un composé selon la revendication 1, qui comprend la réaction d'un composé de formule 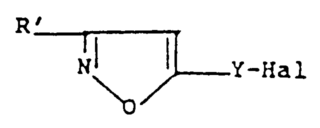 avec un composé de formule 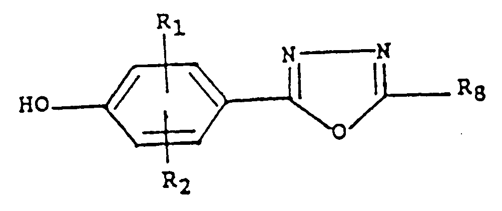 dans laquelle Hal est l'atome de chlore, l'atome de brome ou l'atome d'iode.

17. Procédé de préparation d'un composé selon la revendication 1, qui comprend la réaction d'un composé de formule 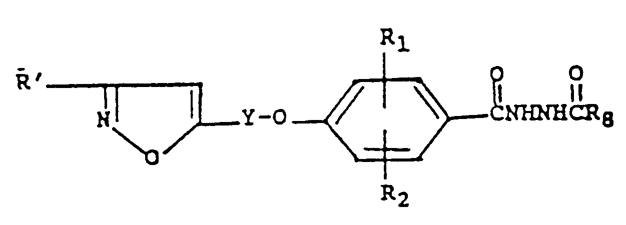 avec de l'hexaméthyldisilazane en présence de trifluorure de tétrabutylammonium.

18. Procédé de préparation d'un composé selon la revendication 1, dans lequel R' est un groupement alkyle en C₁-C₅ et Het est un groupement (alkyle en C₁-C₅)-1,3,4-oxadiazolyle, qui comprend la réaction d'un composé de formule 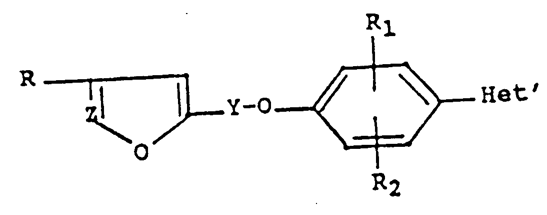 dans laquelle:
Y est un pont alkylène en C₃-C₉,
Z est N,
R est un groupement alkyle en C₁-C₅,
R₁ et R₂ sont un atome d'hydrogène, un atome d'halogène, un groupement alkyle en C₁-C₅, un groupement alcoxy en C₁-C₅, un groupement nitro, un groupement (alcoxy en C₂-C₄)carbonyle ou un groupement trifluorométhyle et
Het' est un groupement 1H-tétrazol-5-yle
avec un chlorure d'acide alcanoïque de formule R₈CO-Hal'' pour lequel Hal'' est l'atome de chlore ou l'atome de brome, ou avec un anhydride d'acide alcanoïque de formule (R₈CO)₂O.

19. Composé selon l'une quelconque des revendications 1 à 7, à utiliser pour contrôler des infections virales.

20. Utilisation d'un composé selon l'une quelconque des revendications 1 à 7 en tant qu'agent antiviral non thérapeutique.

21. Utilisation d'un composé selon l'une quelconque des revendications 1 à 7, destiné à la préparation d'un médicament pour lutter contre des infections virales.

## Revendications (Revendications pour l'(les) Etat(s) contractant(s) suivant(s): ES)

1. Procédé de préparation d'un composé de formule 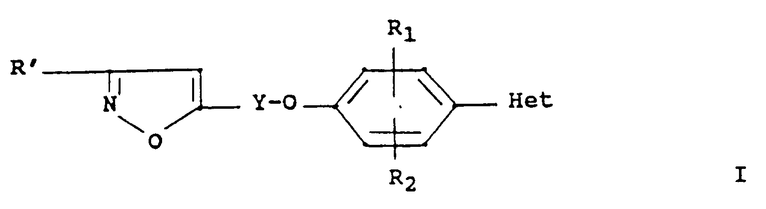 dans laquelle:
Het est choisi parmi 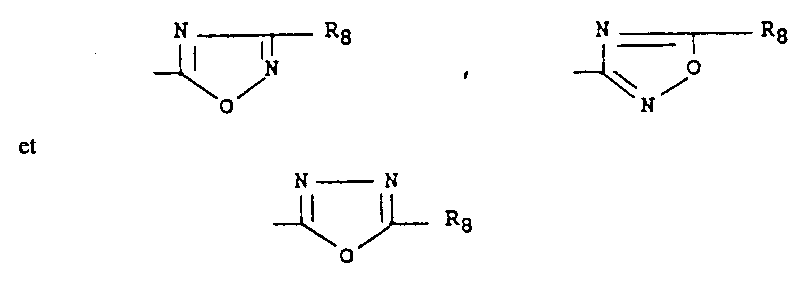 Y est un pont alkylène en C₃-C₉,
R' est un groupement alkyle en C₁-C₅ ou un groupement hydroxyalkyle en C₁-C₅,
R₁ et R₂ sont un atome d'hydrogène, un atome d'halogène, un groupement alkyle en C₁-C₅, un groupement alcoxy en C₁-C₅, un groupement nitro, un groupement (alcoxy en C₁-C₅)carbonyle ou un groupement trifluorométhyle et
R₈ est un groupement alkyle en C₁-C₅,
ou de ses sels d'addition d'acide pharmaceutiquement acceptables par l'une des réactions suivantes:
i) réaction d'un composé de formule 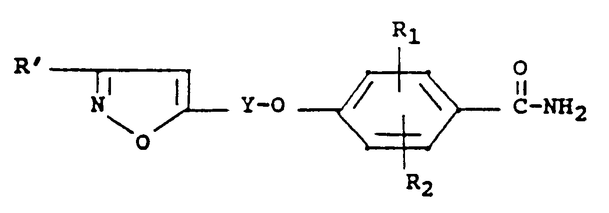 avec un composé de formule (CH₃)₂N-C(OCH₃)₂-R₈, suivie par une réaction du produit résultant avec de l'hydroxylamine en solution d'acide acétique pour produire un composé de formule 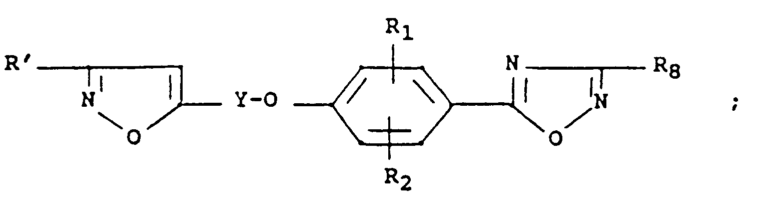
ii) réaction d'un composé de formule 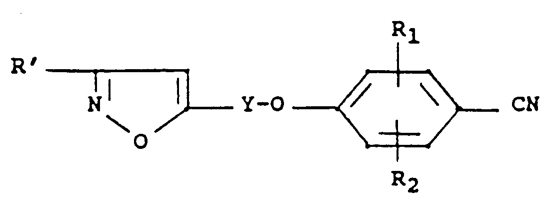 ou de son dérivé de type iminochlorure avec de l'hydroxylamine, suivie par la réaction du produit résultant avec un anhydride de formule (R₈CO)₂O pour produire un composé de formule 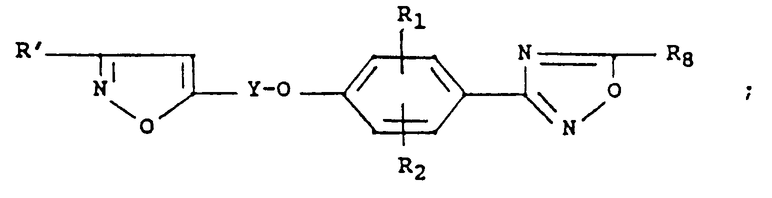
iii) réaction d'un composé de formule 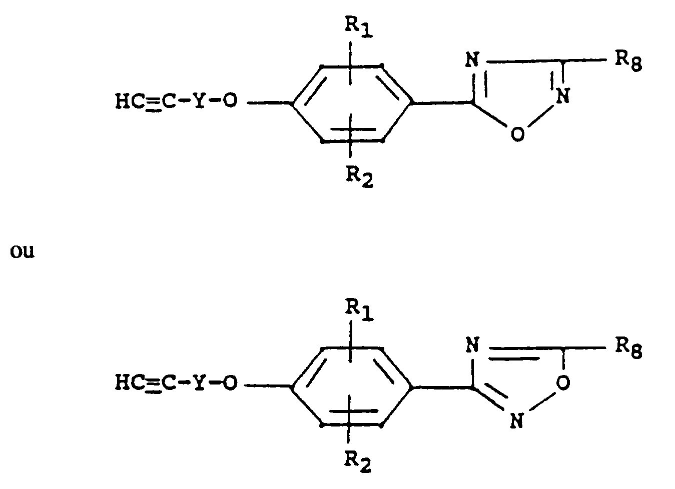 avec un composé de formule R'CH₂NO₂ et du phénylisocyanate,
iv) réaction d'un composé de formule 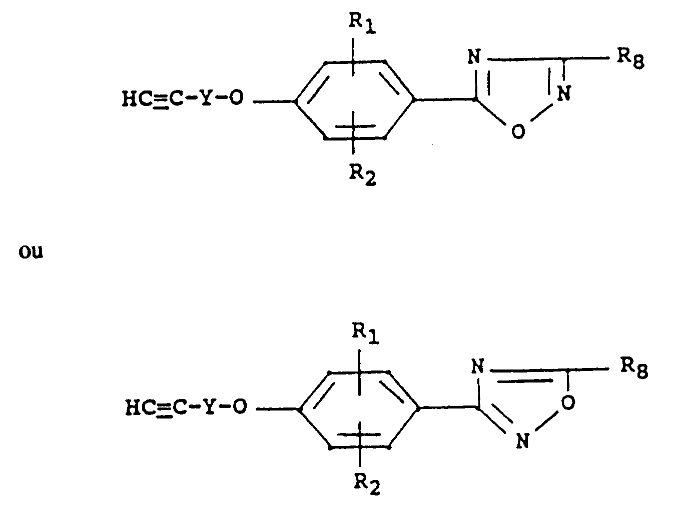 avec un composé de formule R'CH=NOH et avec du N-halogénosuccinimide pour lequel l'atome d'halogène est un atome de brome ou un atome de chlore,
(v) réaction d'un composé de formule 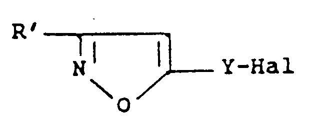 avec un composé de formule 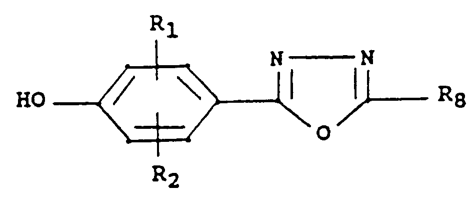 dans laquelle Hal est l'atome de chlore, l'atome de brome ou l'atome d'iode,
(vi) réaction d'un composé de formule 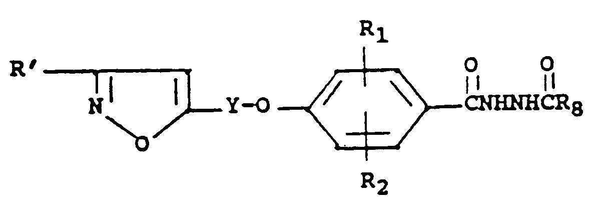 avec de l'hexaméthyldisilazane en présence de trifluorure de tétrabutylammonium ou
(vii) réaction d'un composé de formule 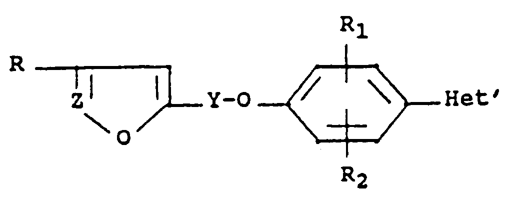 dans laquelle:
Y est un pont alkylène en C₃-C₉,
Z est N,
R est un groupement alkyle en C₁-C₅,
R₁ et R₂ sont un atome d'hydrogène, un atome d'halogène, un groupement alkyle en C₁-C₅, un groupement alcoxy en C₁-C₅, un groupement nitro, un groupement (alcoxy inférieur)carbonyle ou un groupement trifluorométhyle et
Het' est un groupement 1H-tétrazol-5-yle
avec un chlorure d'acide alcanoïque de formule R₈CO-Hal'' pour lequel Hal'' est l'atome de chlore ou l'atome de brome, ou avec un anhydride d'acide alcanoïque de formule (R₈CO)₂O.

2. Procédé selon la revendication 1, dans lequel le produit a la formule 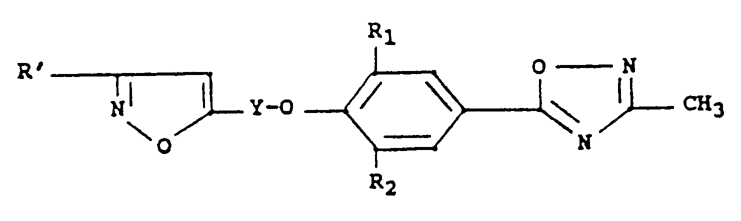 dans laquelle Y est un pont alkylène en C₃-C₉, R' est un groupement alkyle en C₁-C₅ et R₁ et R₂ sont un atome d'hydrogène, un atome d'halogène ou un groupement alkyle en C₁-C₅.

3. Procédé selon la revendication 2, dans lequel le produit est choisi parmi le 5-{5-[2,6-diméthyl-4-(3-méthyl-1,2,4-oxadiazol-5-yl)phénoxy]pentyl}-3-méthylisoxazole, le 5-{3-[2,6-diméthyl-4-(3-méthyl-1,2,4-oxadiazol-5-yl)phénoxy]propyl}-3-méthylisoxazole, le 5-{3-[2,6-diméthyl-4-(3-méthyl-1,2,4-oxadiazol-5-yl)phénoxy]propyl}-3-éthylisoxazole et le 5-{3-[2,6-diméthyl-4-(3-méthyl-1,2,4-oxadiazol-5-yl)phénoxy]propyl}-3-propylisoxazole.

4. Procédé selon la revendication 1, dans lequel le produit a la formule 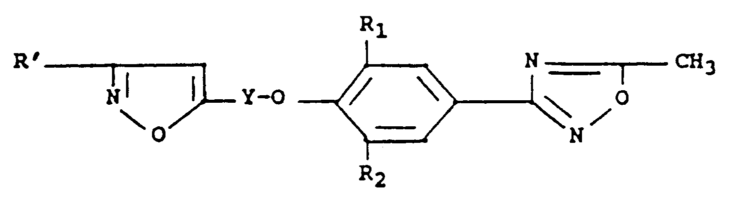 dans laquelle Y est un pont alkylène en C₃-C₉, R' est un groupement alkyle en C₁-C₅ et R₁ et R₂ sont un atome d'hydrogène, un atome d'halogène ou un groupement alkyle en C₁-C₅.

5. Procédé selon la revendication 4, dans lequel le produit est choisi parmi le 5-{5-[2,6-diméthyl-4-(5-méthyl-1,2,4-oxadiazol-3-yl)phénoxy]pentyl}-3-méthylisoxazole, le 5-{3-[2,6-diméthyl-4-(5-méthyl-1,2,4-oxadiazol-3-yl)phénoxy]propyl}-3-méthylisoxazole et le 5-{3-[2,6-diméthyl-4-(5-méthyl-1,2,4-oxadiazol-3-yl)phénoxy]propyl}-3-éthylisoxazole.

6. Procédé selon la revendication 1, dans lequel le produit a la formule 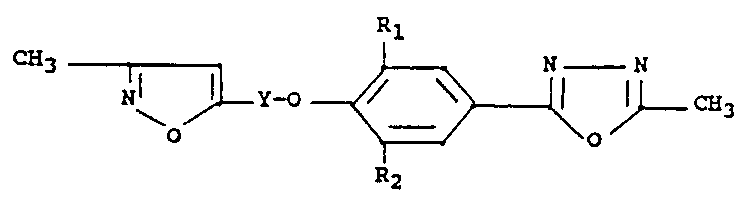 dans laquelle Y, R₁ et R₂ ont les significations données dans la revendication 1.

7. Procédé selon la revendication 6, dans lequel le produit est choisi parmi le 5-{5-[2,6-dichloro-4-(5-méthyl-1,3,4-oxadiazol-2-yl)phénoxy]pentyl}-3-méthylisoxazole et le 5-{3-[2,6-dichloro-4-(5-méthyl-1,3,4-oxadiazol-2-yl)phénoxy]propyl}-3-méthylisoxazole.

8. Utilisation d'un composé selon l'une quelconque des revendications 1 à 7 en tant qu'agent antiviral non thérapeutique.

9. Utilisation d'un composé selon l'une quelconque des revendications 1 à 7, destiné à la préparation d'un médicament pour lutter contre des infections virales.

## Revendications (Revendications pour l'(les) Etat(s) contractant(s) suivant(s): GR)

1. Procédé de préparation d'un composé de formule 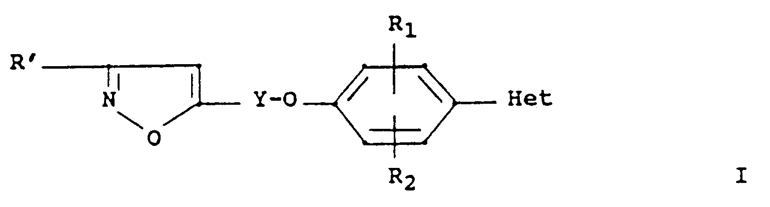 dans laquelle:
Het est choisi parmi 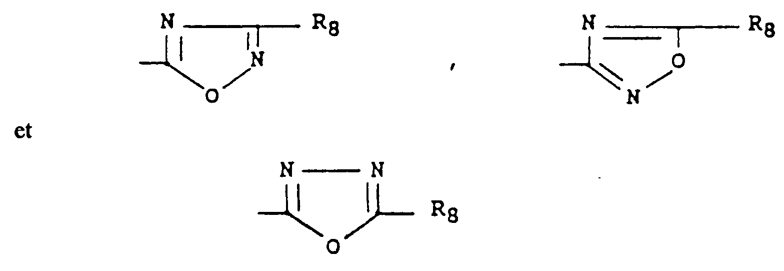 Y est un pont alkylène en C₃-C₉,
R' est un groupement alkyle en C₁-C₅ ou un groupement hydroxyalkyle en C₁-C₅,
R₁ et R₂ sont un atome d'hydrogène, un atome d'halogène, un groupement alkyle en C₁-C₅, un groupement alcoxy en C₁-C₅, un groupement nitro, un groupement (alcoxy en C₁-C₅)carbonyle ou un groupement trifluorométhyle et
R₈ est un groupement alkyle en C₁-C₅,
ou de ses sels d'addition d'acide pharmaceutiquement acceptables par l'une des réactions suivantes:
i) réaction d'un composé de formule 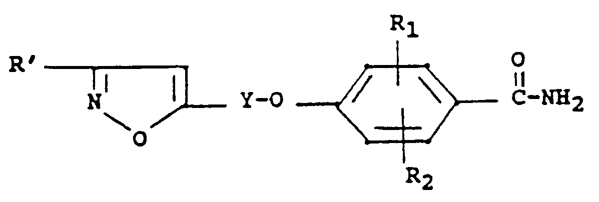 avec un composé de formule (CH₃)₂N-C(OCH₃)₂-R₈, suivie par une réaction du produit résultant avec de l'hydroxylamine en solution d'acide acétique pour produire un composé de formule 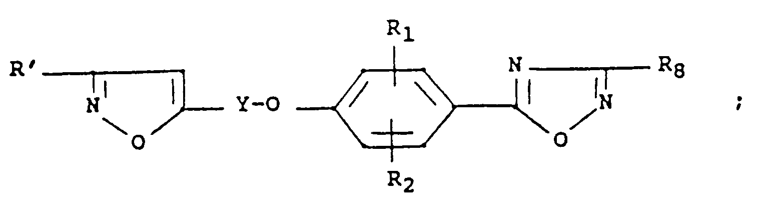
ii) réaction d'un composé de formule 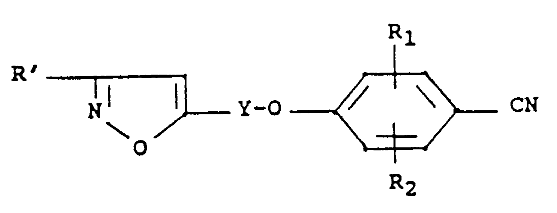 ou de son dérivé de type iminochlorure avec de l'hydroxylamine, suivie par la réaction du produit résultant avec un anhydride de formule (R₈CO)₂O pour produire un composé de formule 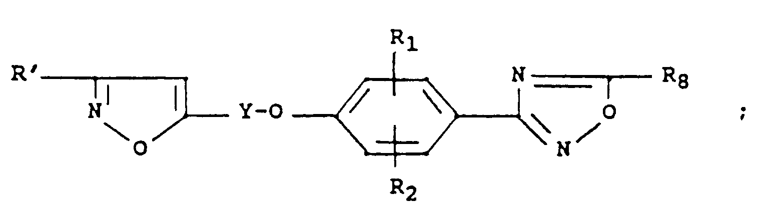
iii) réaction d'un composé de formule 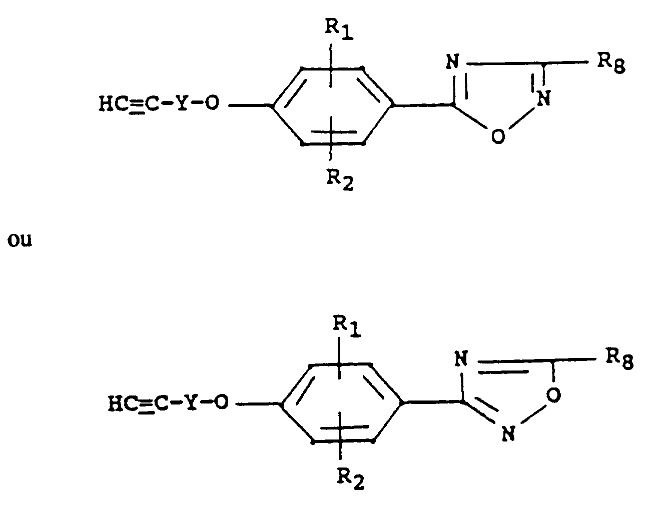 avec un composé de formule R'CH₂NO₂ et du phénylisocyanate,
iv) réaction d'un composé de formule 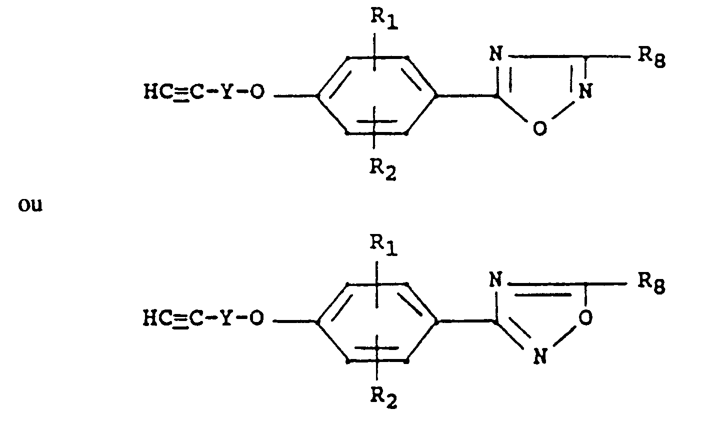 avec un composé de formule R'CH=NOH et avec du N-halogénosuccinimide pour lequel l'atome d'halogène est un atome de brome ou un atome de chlore,
(v) réaction d'un composé de formule 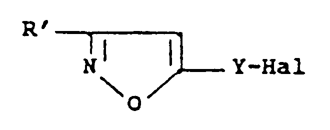 avec un composé de formule 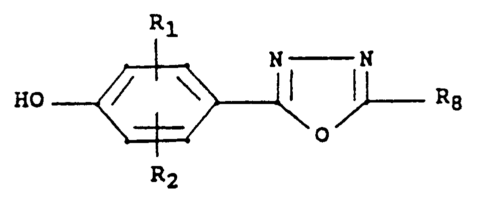 dans laquelle Hal est l'atome de chlore, l'atome de brome ou l'atome d'iode,
(vi) réaction d'un composé de formule 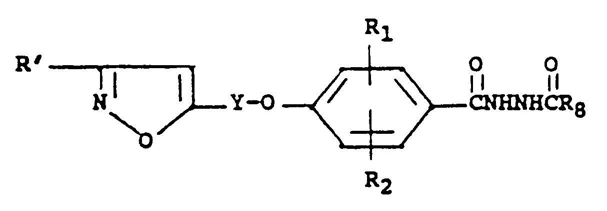 avec de l'hexaméthyldisilazane en présence de trifluorure de tétrabutylammonium ou
(vii) réaction d'un composé de formule 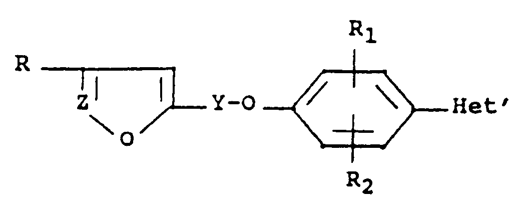 dans laquelle:
Y est un pont alkylène en C₃-C₉,
Z est N,
R est un groupement alkyle en C₁-C₅,
R₁ et R₂ sont un atome d'hydrogène, un atome d'halogène, un groupement alkyle en C₁-C₅, un groupement alcoxy en C₁-C₅, un groupement nitro, un groupement (alcoxy inférieur)carbonyle ou un groupement trifluorométhyle et
Het' est un groupement 1H-tétrazol-5-yle
avec un chlorure d'acide alcanoïque de formule R₈CO-Hal'' pour lequel Hal'' est l'atome de chlore ou l'atome de brome, ou avec un anhydride d'acide alcanoïque de formule (R₈CO)₂O.

2. Procédé selon la revendication 1, dans lequel le produit a la formule 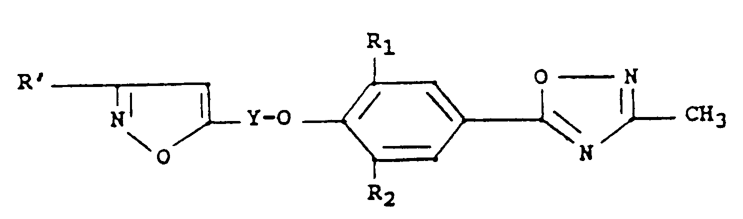 dans laquelle Y est un pont alkylène en C₃-C₉, R' est un groupement alkyle en C₁-C₅ et R₁ et R₂ sont un atome d'hydrogène, un atome d'halogène ou un groupement alkyle en C₁-C₅.

3. Procédé selon la revendication 2, dans lequel le produit est choisi parmi le 5-{5-[2,6-diméthyl-4-(3-méthyl-1,2,4-oxadiazol-5-yl)phénoxy]pentyl}-3-méthylisoxazole, le 5-{3-[2,6-diméthyl-4-(3-méthyl-1,2,4-oxadiazol-5-yl)phénoxy]propyl}-3-méthylisoxazole, le 5-{3-[2,6-diméthyl-4-(3-méthyl-1,2,4-oxadiazol-5-yl)phénoxy]propyl}-3-éthylisoxazole et le 5-{3-[2,6-diméthyl-4-(3-méthyl-1,2,4-oxadiazol-5-yl)phénoxy]propyl}-3-propylisoxazole.

4. Procédé selon la revendication 1, dans lequel le produit a la formule 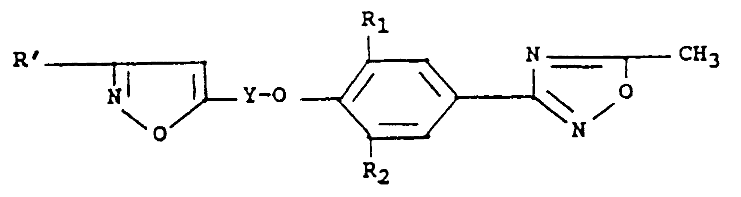 dans laquelle Y est un pont alkylène en C₃-C₉, R' est un groupement alkyle en C₁-C₅ et R₁ et R₂ sont un atome d'hydrogène, un atome d'halogène ou un groupement alkyle en C₁-C₅.

5. Procédé selon la revendication 4, dans lequel le produit est choisi parmi le 5-{5-[2,6-diméthyl-4-(5-méthyl-1,2,4-oxadiazol-3-yl)phénoxy]pentyl}-3-méthylisoxazole, le 5-{3-[2,6-diméthyl-4-(5-méthyl-1,2,4-oxadiazol-3-yl)phénoxy]propyl}-3-méthylisoxazole et le 5-{3-[2,6-diméthyl-4-(5-méthyl-1,2,4-oxadiazol-3-yl)phénoxy]propyl}-3-éthylisoxazole.

6. Procédé selon la revendication 1, dans lequel le produit a la formule 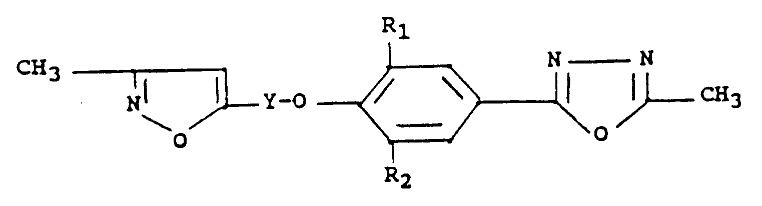 dans laquelle Y, R₁ et R₂ ont les significations données dans la revendication 1.

7. Procédé selon la revendication 6, dans lequel le produit est choisi parmi le 5-{5-[2,6-dichloro-4-(5-méthyl-1,3,4-oxadiazol-2-yl)phénoxy]pentyl}-3-méthylisoxazole et le 5-{3-[2,6-dichloro-4-(5-méthyl-1,3,4-oxadiazol-2-yl)phénoxy]propyl}-3-méthylisoxazole.

8. Utilisation d'un composé selon l'une quelconque des revendications 1 à 7 en tant qu'agent antiviral non thérapeutique.

9. Utilisation d'un composé selon l'une quelconque des revendications 1 à 7, destinée à la préparation d'un médicament pour lutter contre des infections virales.
